# EUROPEAN PATENT SPECIFICATION

(11) **EP 2 069 791 B1**
(45) Date of publication and mention of the grant of the patent: **25.01.2012**
(21) Application number: 07813497.0
(22) Date of filing: 27.07.2007
(51) Int. Cl.: G01N 33/567, A61K 39/395

(54) **CELLULAR RECEPTOR FOR ANTIPROLIFERATIVE FACTOR**
ZELLULÄRER REZEPTOR FÜR ANTIPROLIFERATIVEN FAKTOR
RECEPTEUR CELLULAIRE DU FACTEUR ANTIPROLIFERATION

(30) Priority: 27.07.2006 US 833828 P
(43) Date of publication of application: 17.06.2009
(73) Proprietor: University of Maryland, Baltimore, Baltimore MD 21201 (US); Government of the United States of America as represented by The Secretary of the Department of Health and Human Services, Rockville, MD 20852-3804 (US)
(72) Inventor: KEAY, Susan, K., Ellicott City, MD 21042 (US); MICHEJDA, Christopher, North Potomac, MD 20878 (US); TOCCI, Gillian, Hanover, PA 18109 (US); CONRADS, Thomas, Pittsburgh, PA 15217 (US); VEENSTRA, Timothy, Jefferson, MD 21755 (US); HOOD, Brian, Lewis, Pittsburgh, Pennsylvania 15237 (US)
(74) Representative: Woods, Geoffrey Corlett
(86) International application number: PCT/US2007/074643
(87) International publication number: WO 2008/014484

(56) References cited:
- US-A- 5 523 290
- US-A- 5 962 645
- US-A- 5 962 645
- US-A1- 2002 016 443
- US-A1- 2005 096 263

### SEQUENCE LISTING

<110> Keay, Susan
   Michejda, Christopher
   Hussey, Gillian
   Conrads, Thomas
   Veenstra, Timothy
<120> CELLULAR RECEPTOR FOR ANTIPROLIFERATIVE FACTOR
<130> HO-PO3302WOO
<140> Not Assigned
   <141> 2007-07-27
<150> US 60/833,828 <151> 2006-07-27
<160> 13
<170> PatentIn version 3.3
<210> 1
   <211> 9
   <212> PRT
   <213> ARTIFICIAL SEQUENCE
<220>
   <223> Synthetic Peptide
<400> 1
   Thr Val Pro Ala Ala Val Val Val Ala
   1               5
<210> 2
   <211> 9

   <212> PRT
   <213> ARTIFICIAL SEQUENCE
<220>
   <223> Synthetic Peptide
<400> 2
   Ser Val Pro Ala Ala Val Val Val Ala
   1               5
<210> 3
   <211> 9
   <212> PRT
   <213> ARTIFICIAL SEQUENCE
<220>
   <223> Synthetic Peptide
<400> 3
   Thr Val Pro Ala Ala Val Val Leu Ala
   1 5
<210> 4
   <211> 9
   <212> PRT
   <213> ARTIFICIAL SEQUENCE
<220>
   <223> Synthetic Peptide
<400> 4
   Ser Leu Pro Ala Ala Val Val Val Ala
   1 5
<210> 5
   <211> 23
   <212> RNA
   <213> Artificial sequence
<220>
   <223> Oligonucleotide
<400> 5
   aacuuuugag uccaucuuga gaa 23
<210> 6
   <211> 522
   <212> PRT
   <213> Human
<400> 6
<210> 7
   <211> 2231
   <212> DNA
   <213> Human
<400> 7
   agccaatggg gctcgctcgc ctcccagccc gcggcccgag ccgccgccgc gcccgccatg 60
   ccctcggcca aacaaagggg ctccaagggc ggccacggcg ccgcgagccc ctcggagaag 120
   ggtgcccacc cgtcgggcgg cgcggatgac gtggcgaaga agccgccgcc ggcgccgcag 180
   cagccgccgc cgccgcccgc gccgcacccg cagcagcacc cgcagcagca cccgcagaac 240
   caggcgcacg gcaagggcgg ccaccgcggc ggcggcggcg gcggcggcaa gtcctcctcc 300
   tcctcctccg cctccgccgc cgctgccgcc gccgccgcct cgtcctcggc gtcctgctcg 360
   cgcaggctcg gcagggcgct caactttctc ttctacctcg ccctggtggc ggcggccgct 420
   ttctcgggct ggtgcgtcca ccacgtcctg gaggaggtcc agcaggtccg gcgcagccac 480
   caggacttct cccggcagag ggaggagctg ggccagggct tgcagggcgt cgagcagaag 540
   gtgcagtctt tgcaagccac atttggaact tttgagtcca tcttgagaag ctcccaacat 600
   aaacaagacc tcacagagaa agctgtgaag caaggggaga gtgaggtcag ccggatcagc 660
   gaagtgctgc agaaactcca gaatgagatt ctcaaagacc tctcggatgg gatccatgtg 720
   gtgaaggacg cccgggagcg ggacttcacg tccctggaga acacggtgga ggagcggctg 780
   acggagctca ccaaatccat caacgacaac atcgccatct tcacagaagt ccagaagagg 840
   agccagaagg agatcaatga catgaaggca aaggttgcct ccctggaaga atctgagggg 900
   aacaagcagg atttgaaagc cttaaaggaa gctgtgaagg agatacagac ctcagccaag 960
   tccagagagt gggacatgga ggccctgaga agtacccttc agactatgga gtctgacatc 1020
   tacaccgagg tccgcgagct ggtgagcctc aagcaggagc agcaggcttt caaggaggcg 1080
   gccgacacgg agcggctcgc cctgcaggcc ctcacggaga agcttctcag gtctgaggag 1140
   tccgtctccc gcctcccgga ggagatccgg agactggagg aagagctccg ccagctgaag 1200
   tccgattccc acgggccgaa ggaggacgga ggcttcagac actcggaagc ctttgaggca 1260
   ctccagcaaa agagtcaggg actggactcc aggctccagc acgtggagga tggggtgctc 1320
   tccatgcagg tggcttctgc gcgccagacc gagagcctgg agtccctcct gtccaagagc 1380
   caggagcacg agcagcgcct ggccgccctg caggggcgcc tggaaggcct cgggtcctca 1440
   gaggcagacc aggatggcct ggccagcacg gtgaggagcc tgggcgagac ccagctggtg 1500
   ctctacggtg acgtggagga gctgaagagg agtgtgggcg agctccccag caccgtggaa 1560
   tcactccaga aggtgcagga gcaggtgcac acgctgctca gtcaggacca agcccaggcc 1620
   gcccgtctgc ctcctcagga cttcctggac agactttctt ctctagacaa cctgaaagcc 1680
   tcagtcagcc aagtggaggc ggacttgaaa atgctcagga ctgctgtgga cagtttggtt 1740
   gcatactcgg tcaaaataga aaccaacgag aacaatctgg aatcagccaa gggtttacta 1800
   gatgacctga ggaatgatct ggataggttg tttgtgaaag tggagaagat tcacgaaaag 1860
   gtctaaatga attgcgtgtg cagggcgcgg atttaaagtc caatttctca tgaccaaaaa 1920
   atgtgtggtt ttttcccatg tgtcccctac cccccaattt cttgtcccct cttaaagagc 1980
   agttgtcacc acctgaacac caaggcattg tattttcatg cccagttaac ttatttacaa 2040
   tatttaagtt ctctgcttct gcatttggtt ggtttcctga agcgcagccc ctgtgaataa 2100
   caggtggctt ttcatggatg tctctagtca gagaaaaatg ataaaggctt aaattgagga 2160
   ttaacagaag cagattaacc tcagaaatcc tgtctggctg gcagatttca agtaaaaaaa 2220
   aaaaaaaaaa a 2231
<210> 8
   <211> 466
   <212> PRT <213> Human
<400> 8
   Met Ser Thr Arg Ser Val Ser Ser Ser Ser Tyr Arg Arg Met Phe Gly
   1               5                   10                  15
   Gly Pro Gly Thr Ala Ser Arg Pro Ser Ser Ser Arg Ser Tyr Val Thr
               20                  25                  30
   Thr Ser Thr Arg Thr Tyr Ser Leu Gly Ser Ala Leu Arg Pro Ser Thr
           35                  40                  45
   Ser Arg Ser Leu Tyr Ala Ser Ser Pro Gly Gly Val Tyr Ala Thr Arg
       50                  55                  60
   Ser Ser Ala Val Arg Leu Arg Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
   450 455 460
   Leu Glu
   465
<210> 9 <211> 1749
   <212> DNA
   <213> Human
<400> 9
   gagggcgccc ccaccccacc cgcccaccct ccccgcttct cgctaggtcc cgattggctg 60
   gcgcgctccg cggctgggat ggcagtggga ggggaccctc tttcctaacg gggttataaa 120
   aacagcgccc tcggcggggt ccagtcctct gccactctcg ctccgaggtc cccgcgccag 180
   agacgcagcc gcgctcccac cacccacacc caccgcgccc tcgttcgcct cttctccggg 240
   agccagtccg cgccaccgcc gccgcccagc ccatcgccac cctccgcagc catgtccacc 300
   aggtccgtgt cctcgtcctc ctaccgcagg atgttcggcg gcccgggcac cgcgagccgg 360
   ccgagctcca gccggagcta cgtgactacg tccacccgca cctacagcct gggcgacgcg 420
   ctgcgcccca gcaccagccg cagcctctac gcctcgtccc cgggcggcgt gtatgccacg 480
   cgctcctctg ccgtgcgcct gcggagcagc gtgcccgggg tgcggctcct gcaggactcg 540
   gtggacttct cgctggccga cgccatcaac accgagttca agaacacccg caccaacgag 600
   aaggtggagc tgcaggagct gaatgaccgc ttcgccaact acatcgacaa ggtgcgcttc 660
   ctggagcagc agaataagat cctgctggcc gagctcgagc agctcaaggg ccaaggcaag 720
   tcgcgcctag gggacctcta cgaggaggag atgcgggagc tgcgccggca ggtggaccag 780
   ctaaccaacg acaaagcccg cgtcgaggtg gagcgcgaca acctggccga ggacatcatg 840
   cgcctccggg aaaaattgca ggaggagatg cttcagagag aggaagccga aaacaccctg 900
   caatctttca gacaggatgt tgacaatgcg tctctggcac gtcttgacct tgaacgcaaa 960
   gtggaatctt tgcaagaaga gattgccttt ttgaagaaac tccacgaaga ggaaatccag 1020
   gagctgcagg ctcagattca ggaacagcat gtccaaatcg atgtggatgt ttccaagcct 1080
   gacctcacgg ctgccctgcg tgacgtacgt cagcaatatg aaagtgtggc tgccaagaac 1140
   ctgcaggagg cagaagaatg gtacaaatcc aagtttgctg acctctctga ggctgccaac 1200
   cggaacaatg acgccctgcg ccaggcaaag caggagtcca ctgagtaccg gagacaggtg 1260
   cagtccctca cctgtgaagt ggatgccctt aaaggaacca atgagtccct ggaacgccag 1320
   atgcgtgaaa tggaagagaa ctttgccgtt gaagctgcta actaccaaga cactattggc 1380
   cgcctgcagg atgagattca gaatatgaag gaggaaatgg ctcgtcacct tcgtgaatac 1440
   caagacctgc tcaatgttaa gatggccctt gacattgaga ttgccaccta caggaagctg 1500
   ctggaaggcg aggagagcag gatttctctg cctcttccaa acttttcctc cctgaacctg 1560
   agggaaacta atctggattc actccctctg gttgataccc actcaaaaag gacattcctg 1620
   attaagacgg ttgaaactag agatggacag gttatcaacg aaacttctca gcatcacgat 1680
   gaccttgaat aaacaattgc acactcagtg cagcactcat ataccagcag ataaaagaat 1740
   ccatatctt 1749
<210> 10
   <211> 8
   <212> PRT
   <213> Artificial sequence
<220>
   <223> Synthetic Peptide
<400> 10
   Thr Val Pro Ala Ala Val Val Ala
   1               5
<210> 11
   <211> 602
   <212> PRT
   <213> Human
<400> 11
<210> 12
   <211> 2913
   <212> DNA
   <213> Human
<400> 12
   gggggagccc ctgcaagttt cccgggccgc gcgccgcgct cgctcgcctc ccagcccgcg      60
   gcccgagccg ccgccgcgcc cgccatgccc tcggccaaac aaaggggctc caagggcggc     120
   cacggcgccg cgagcccctc ggagaagggt gcccacccgt cgggcggcgc ggatgacgtg     180
   gcgaagaagc cgccgccggc gccgcagcag ccgccgccgc cgcccgcgcc gcacccgcag     240
   cagcacccgc agcagcaccc gcagaaccag gcgcacggca agggcggcca ccgcggcggc 300
   ggcggcggcg gcggcaagtc ctcctcctcc tcctccgcct ccgccgccgc tgccgccgcc 360
   gccgcctcgt cctcggcgtc ctgctcgcgc aggctcggca gggcgctcaa ctttctcttc 420
   tacctcgccc tggtggcggc ggccgctttc tcgggctggt gcgtccacca cgtcctggag 480
   gaggtccagc aggtccggcg cagccaccag gacttctccc ggcagaggga ggagctgggc 540
   cagggcttgc agggcgtcga gcagaaggtg cagtctttgc aagccacatt tggaactttt 600
   gagtccatct tgagaagctc ccaacataaa caagacctca cagagaaagc tgtgaagcaa 660
   ggggagagtg aggtcagccg gatcagcgaa gtgctgcaga aactccagaa tgagattctc 720
   aaagacctct cggatgggat ccatgtggtg aaggacgccc gggagcggga cttcacgtcc 780
   ctggagaaca cggtggagga gcggctgacg gagctcacca aatccatcaa cgacaacatc 840
   gccatcttca cagaagtcca gaagaggagc cagaaggaga tcaatgacat gaaggcaaag 900
   gttgcctccc tggaagaatc tgaggggaac aagcaggatt tgaaagcctt aaaggaagct 960
   gtgaaggaga tacagacctc agccaagtcc agagagtggg acatggaggc cctgagaagt 1020
   acccttcaga ctatggagtc tgacatctac accgaggttc gcgagctggt gagcctcaag 1080
   caggagcagc aggctttcaa ggaggcggcc gacacggagc ggctcgccct gcaggccctc 1140
   acggagaagc ttctcaggtc tgaggagtcc gtctcccgcc tcccggagga gatccggaga 1200
   ctggaggaag agctccgcca gctgaagtcc gattcccacg ggccgaagga ggacggaggc 1260
   ttcagacact cggaagcctt tgaggcactc cagcaaaaga gtcagggact ggactccagg 1320
   ctccagcacg tggaggatgg ggtgctctcc atgcaggtgg cttctgcgcg ccagaccgag 1380
   agcctggagt ccctcctgtc caagagccag gagcacgagc agcgcctggc cgccctgcag 1440
   gggcgcctgg aaggcctcgg gtcctcagag gcagaccagg atggcctggc cagcacggtg 1500
   aggagcctgg gcgagaccca gctggtgctc tacggtgacg tggaggagct gaagaggagt 1560
   gtgggcgagc tccccagcac cgtggaatca ctccagaagg tgcaggagca ggtgcacacg 1620
   ctgctcagtc aggaccaagc ccaggccgcc cgtctgcctc ctcaggactt cctggacaga 1680
   ctttcttctc tagacaacct gaaagcctca gtcagccaag tggaggcgga cttgaaaatg 1740
   ctcaggactg ctgtggacag tttggttgca tactcggtca aaatagaaac caacgagaac 1800
   aatctggaat cagccaaggg tttactagat gacctgagga atgatctgga taggttgttt 1860
   gtgaaagtgg agaagattca cgaaaaggtc taaatgaatt gcgtgtgcag ggcgcggatt 1920
   taaagtccaa tttctcatga ccaaaaaatg tgtggttttt tcccatgtgt cccctacccc 1980
   ccaatttctt gtcccctctt aaagagcagt tgtcaccacc tgaacaccaa ggcattgtat 2040
   tttcatgccc agttaactta tttacaatat ttaagttctc tgcttctgca tttggttggt 2100
   ttcctgaagc gcagcccctg tgaataacag gtggcttttc atggatgtct ctagtcagag 2160
   aaaaatgata aaggcttaaa ttgaggatta acagaagcag attaacctca gaaatcctgt   2220
   ctggctggca gatttcaagt aaaaaaaaaa aaaaggtggg ttggggggac ccttttcttt   2280
   ctagttgtct ttaaggaaaa ttaattttac tttttttttt gttctggccg aaatttttat   2340
   gagatatctc tcacttgtct tccactttga accggttaaa gctcatagct gtcagctctg   2400
   aatgaggagg ggagaagccc ctgggtcttt ctttgaaagg aatccgctgc ttgagggctg   2460
   cctccctcat ggtgtgcgtg tcgttctctt cctgacgcat ctgtgatatc agaggtaact   2520
   atgcaaagca tccaggcggt tctgaatgtg aagcactaca cccagcagag tcccggtgcc   2580
   ctctgtcccc actgccggcc catgtcctct ctccggaggt caccaaggaa tgcacaggtt   2640
   tcgactacca gaaaggggag tccttgggtt ctttcaaaaa attcgtgagg agagctgtct   2700
   acagtggaat agggggtctc cctggggaat gcaggccaag tccttttatt ttaacatgat   2760
   gtccatgaag aggtttgccg tctgggcagc cctgtcggca aggagcgtgc atactgcgtt   2820
   tgtgtaattg tttgctgtat ctcccttccc tctgagctgt attgttcttt aatggctgtc   2880
   ttgcccttcc aaaaaaaatt gaaaaaaaaa aaa                                2913
<210> 13
   <211> 23
   <212> RNA
   <213> Artificial Sequence
<220>
   <223> Synthetic RNA oligonucleotide
<400> 13
   aauucuguau gcuaccugua gaa 23

## Description

### FIELD OF THE INVENTION

The present invention concerns at least the fields of molecular biology, cellular biology, and medicine. In general embodiments, the present invention concerns the field of urology and/or cell regulation, and in particular embodiments it is directed to at least interstitial cystitis and/or cancer.

### BACKGROUND OF THE INVENTION

Antiproliferative factor (APF) is a sialoglycopeptide inhibitor of bladder epithelial cell proliferation that is at least secreted specifically by bladder epithelial cells from patients with interstitial cystitis (IC) (Keay *et al.,* 2004; Keay *et al.,* 2000) a disorder commonly associated with denudation or thinning of the bladder epithelium (Skoluda *et al*., 1974; Matthews *et al*., 2001; Held *et al.,* 1990). APF was discovered to be the active factor in urine from IC patients that reversibly inhibited the growth of bladder epithelial cells *in vitro* (Keay *et al*., 2000; Keay *et al.,* 1996). The specificity of APF for urine from IC patients (vs. normal controls or patients with a variety of other urogenital disorders (Keay *et al*., 2001)) indicates that in certain aspects it is useful as a diagnostic marker for IC and that it may play an important role in the pathogenesis of this disorder.

APF is the first naturally occurring, low molecular weight negative growth regulator to have been identified and completely characterized. The peptide sequence of APF is identical to residues 541-549 of the 6th transmembrane domain of Frizzled 8, a Wnt ligand receptor. The glycosyl moiety of APF consists of sialic acid α-2,3 linked to galactose β1-3-N-acetylgalactosamine, which is α-O-linked to the N-terminal threonine residue of the nonapeptide 1.

APF has been shown to profoundly inhibit the proliferation of both normal bladder epithelial cells and bladder carcinoma cells *in vitro* (Keay *et al.,* 2004; Keay *et al.,* 2000; Keay *et al*., 1996). Furthermore, APF can induce multiple changes in the pattern of cellular gene expression including decreased production of heparin-binding epidermal growth factor-like growth factor (HB-EGF) and increased production of E-cadherin, resulting in a more differentiated bladder epithelial cell phenotype (Keay *et al*., 2000; Keay *et al.,* 2003). APF was also recently determined to decrease tight junction protein (zonula occludens- and occludin) production and increase paracellular permeability of normal bladder epithelial cell monolayers similar to changes seen in cells from patients with IC *in vitro* (Zhang *et al.,* 2005).

The potency of APF (EC₅₀ in the picomolar range), its varied effects on bladder epithelial cell protein expression and proliferation, and the requirement for a hexosamine-galactose disaccharide linked in a specific alpha configuration to the backbone peptide for activity (Keay *et al.,* 2004; Keay *et al.,* 2000; Zhang *et al.,* 2005), all indicate that APF's effects are likely to be mediated by binding to and activating a receptor, for example. Microarray analysis indicated that there may be a role for specific transcription factors, such as AP-1, SP-1 and TCF/LEF-1, in abnormal gene expression in cells explanted from IC patients or following APF treatment of normal cells; this provides additional evidence for involvement of a receptor (Keay *et al.,* 2003). Identification of a receptor for APF is important for understanding its mechanism of action, and in certain embodiments of the invention leads to development of therapeutic agents for IC that specifically bind to APF and/or its receptor to block its effects on the bladder epithelium. US 5,962,645 and US 2002/ 006443 disclose screening methods using APF.

### SUMMARY OF THE INVENTION

According to the present invention, there is provided a method of screening for a modulator of the binding of APF to cytoskeletal associated protein 4 (CKAP4), comprising:
(a) providing a candidate modulator;
(b) admixing the candidate modulator with APF, or an antiproliferative congener of APF, and CKAP4;
(c) measuring binding of APF to CKAP4; and
(d) comparing the binding measured in step (c) with the binding in the absence of said candidate modulator, wherein a difference indicates that said candidate modulator is a modulator of the binding of APF to CKAP4.
   The modulator may enhance binding of APF to CKAP4. Alternatively, the modulator may inhibit binding of APF to CKAP4. Binding of the candidate modulator to CKAP4 can be measured in step (c).
   The method may further comprise formulating said modulator for delivery to an individual in need thereof. The individual may be an individual with interstitial cystitis and the modulator may be an inhibitor of the binding of APF to the CKAP4. Alternatively, the individual may have cancer or at least one risk factor for developing bladder cancer or kidney cancer and the modulator may enhance binding of APF to CKAP4.
   The method may further comprise the step of assaying the candidate modulator for cell growth inhibition activity.

Antiproliferative factor (APF) is a frizzled-8 protein-related low molecular mass glycopeptide that is made by bladder epithelial cells from patients with interstitial cystitis (IC). Because epithelial thinning or ulceration are the most common histologic abnormalities in IC, APF contributes to the pathogenesis of this disorder. The present invention concerns identification of a bladder epithelial cell receptor for APF (the receptor is cytoskeleton associated protein 4 (CKAP4)/p63, identified herewith). The invention allows for development of agents that bind to APF or its receptor and modulate the activity of APF, the activity of the APF receptor, or both. In particular embodiments, there are compounds employed that inhibit APF binding to its receptor. In specific embodiments, the modulation relates to the inhibition of the activity or enhancement of the activity. Such agents, particularly at least those that inhibit APF activity, inhibit APF receptor activity, or APF binding to its receptor, are employed to treat IC, as an exemplary bladder condition. In addition, identification of this receptor facilitates the development of one or more agents that are useful for treatment of interstitial cystitis. In specific embodiments, the one or more agents inhibit pathways activated by APF binding to an APF receptor, and these agents are useful at least for treatment of IC, in certain aspects. In certain embodiments, APF is also a potent inhibitor of bladder cancer cell growth. Therefore, a receptor, receptor variant, or modified variant of APF, such as one identified by assays employing APF receptor or the knowledge thereof, are employed to identify compounds for the treatment of carcinomas or other hyperproliferative disorders, for example. In a specific embodiment, the compounds are agonists for APF.

In particular, identification of the APF receptor allows for development of agents that bind to APF and/or the receptor and inhibit APF activity, or APF receptor activity, or agents that inhibit signaling pathways activated by APF binding to its receptor; certain of these agents are useful for IC treatment. Also, the receptor or a modified variant thereof is useful for detection of APF, thereby providing a means for a diagnostic test for IC, and it may be detected in urine, biopsy, solid tissue, serum samples, plasma samples, saliva or any other bodily fluid, or combinations thereof, for example. In addition, the receptor or a modified variant thereof are used in one or more high throughput assays to identify agents that inhibit APF production, and these agents may also be employed at least as an IC treatment. Finally, the receptor or a modified variant thereof is useful to identify agonists for APF for the treatment of carcinomas or other hyperproliferative disorders.

In specific aspects of the invention, the mode of binding for APF to the receptor, and particularly to the APF receptor (which comprises at least CKAP4/p63), is determined. Agents including synthetic agents, natural agents, or combinations thereof, that mimic the binding site on CKAP4/p63 may also be developed, such as by standard means in the art, for example, and this may include determination of the ability of these agents to inhibit APF biological activity. An agent that mimics the binding site on the APF receptor may act as an inhibitor for the binding of APF to its receptor, in certain embodiments of the invention. Such an agent may be employed for treatment or prevention of a bladder condition, such as IC.

The receptor may be employed in any suitable method related to the invention. For example, the receptor may be employed in a clinical application, such as in a method to treat and/or prevent a medical condition in an individual, such as a mammal, and including a human. In particular embodiments, the receptor is utilized in methods to treat and/or prevent a bladder condition, such as, for example, interstitial cystitis. In particular aspects, a compound that inhibits APF, inhibits APF receptor, and/or inhibits binding of APF to the receptor may be employed for treating and/or preventing a bladder condition.

In other embodiments, concerning a hyperproliferative disease such as cancer, compositions include compounds that enhance binding of APF to its receptor and also compounds that comprise expression constructs for expressing the receptor in a cell, thereby rendering the cell more sensitive to APF. The individual being treated may be referred to as having a hyperproliferative disease or as being at risk for developing a hyperproliferative disease. Exemplary hyperproliferative diseases include bladder cancer and/or kidney cancer. For example, an individual at risk for developing bladder cancer may smoke; be exposed to occupational hazards, such as chemicals, including, for example, certain industrial chemicals that have been linked with bladder cancer, such as aromatic amines, including benzidine and beta-naphthylamine; are caucasian; are between 65 and 85 years old; are men; have a history of chronic bladder inflammation; have a history of bladder cancer; have a history of bladder birth defects; have low fluid consumption; and/or exposed to arsenic in drinking water. Also for example, an individual at risk for developing kidney cancer may be smokers; obese; have a sedentary lifestyle; have exposure to asbestos, cadmium (a type of metal), some herbicides, benzene, and organic solvents, particularly trichloroethylene; have genetic and hereditary risk factors; have a family history; and so forth.

In certain aspects the receptor employed for therapeutic, preventative, diagnostic, and/or screening applications is in a soluble form. In other certain aspects, the receptor or a variant thereof acts to modulate, such as inhibit at least in part, APF function or activity, thereby either indirectly or directly treating a bladder condition. Thus, the APF receptor may be considered and/or utilized as an APF inhibitor. The receptor may be employed in combination with another therapy for the individual, including another bladder condition therapy.

In certain aspects, the interstitial cystitis is hereditary and APF and/or its receptor is indicative of susceptibility to development of interstitial cystitis. In other embodiments, production of APF precedes development of interstitial cystitis, including hereditary interstitial cystitis, and APF receptor or a receptor variant is employed to prevent or delay onset of interstitial cystitis.

Any compositions including APF receptor, APF receptor inhibitor, and/or APF receptor agonist, may comprise one or more polypeptides, polynucleotides, small molecules, lipids, sugars, and so forth.

In particular, a receptor for APF as described herein was isolated based on exemplary isolation methods. In other embodiments, at least one of the polypeptides identified in the isolation method is further characterized as an APF receptor by preincubating normal bladder epithelial cells with antibodies against the protein (or an unrelated isotype control antibody) prior to incubation with APF and performance of the ³H-thymidine incorporation cell proliferation assay. In aspects wherein the putative receptor protein is available in a recombinant form, active APF is also preincubated with the receptor protein to further characterize its inhibitory activity toward APF activity in the biological proliferation assay. Inhibition of APF may result from direct binding of APF to the receptor, such as *via* one or more domains on the receptor.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawings.

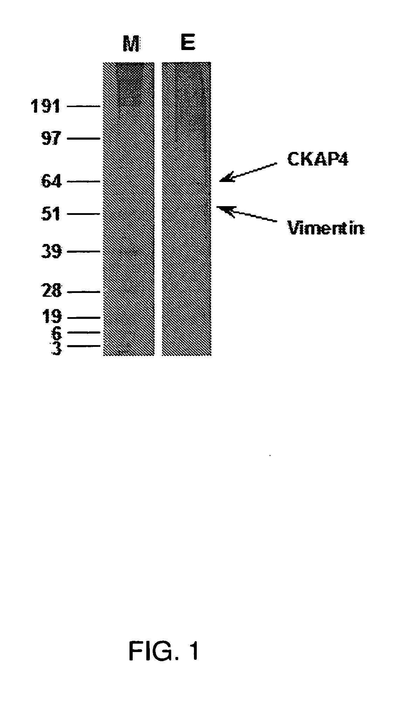

FIGS. 1A-1B demonstrate the isolation of bladder epithelial cell proteins with high affinity binding to APF. In FIG. 1A, there is silver-stained polyacrylamide gel resolution of eluted proteins shown in E; molecular weight markers shown in M. In FIG. 1B, exemplary peptides identified from tryptic digestss of the 63 and 54 kDa bands using nanoRPLC-MS/MS are provided.

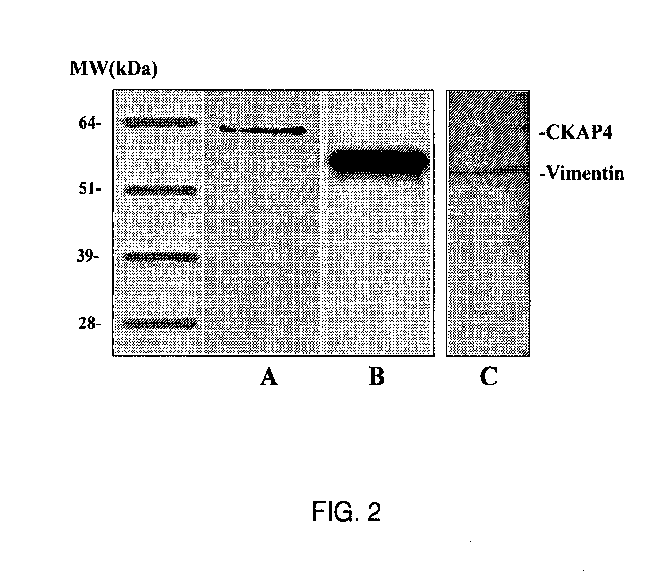

FIGS. 2A-2C show confirmation of identification of CKAP4/p63 and vimentin by Western blot. In FIG. 2A, there is incubation of membranes containing eluted proteins using antibodies against CKAP4/p63. In FIG. 2B, there is incubation of the same eluted proteins with antibodies against vimentin. In FIG. 2C, there is silver stained gel of eluted proteins showing the 63 kDa and 54 kDa bands.

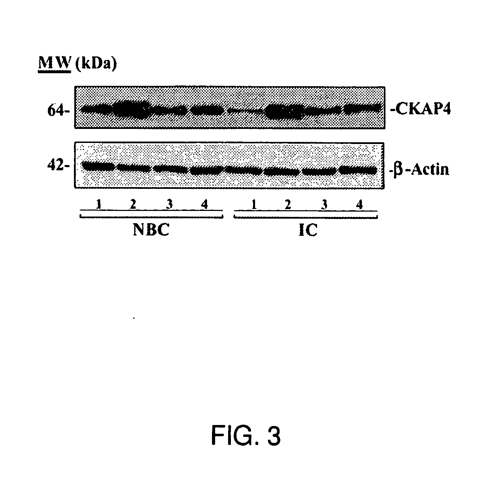

FIG. 3 provides western blot identification of CKAP4/p63 in membrane proteins extracted from bladder epithelial explants obtained from 4 IC patients and their 4 age-, race- and gender-matched normal controls. NBC = normal bladder cells; IC = cells from IC patients.

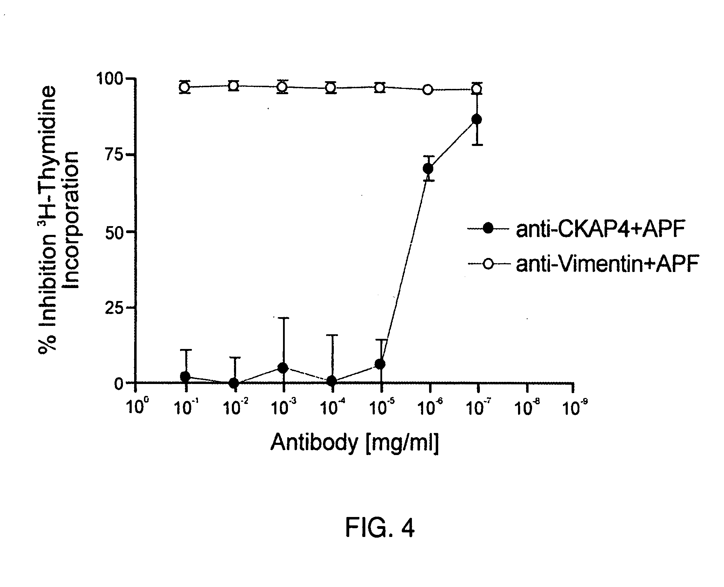

FIG. 4 demonstrates decreased sensitivity of normal bladder cells to APF following preincubation with anti-CKAP4/p63 antibodies.

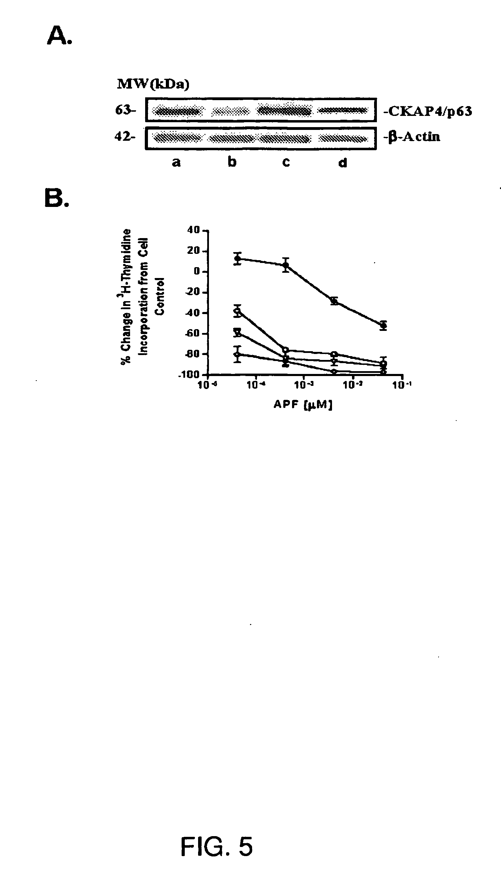

FIGS. 5A-5B show siRNA knockdown of CKAP4/p63 in normal bladder epithelial cells. Western blot of CKAP4/p63 protein expression normalized to beta actin expression in normal bladder epithelial cells (FIG. 5A) following electroporation with double-stranded siRNA to CKAP4/p63 (A); following electroporation procedure alone (without siRNA) (B); receiving no treatment (cell controls) (C). In FIG. 5B, there is decreased sensitivity to APF following electroporation with double-stranded siRNA to CKAP4/p63 -●-; first negative control (electroporation without siRNA) -○- ; second negative control (no electroporation or siRNA) -∇-.

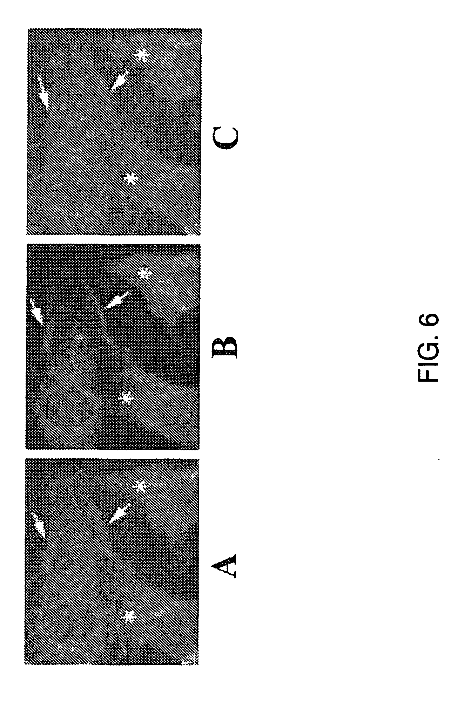

FIGS. 6A-6C provide confocal microscopy of anti-CKAP4/p63 and rhodamine-labeled APF binding in normal bladder epithelial cells. In FIG. 6A, there is binding of anti-CKAP4/p63 followed by FITC-labeled anti-mouse antibodies; in FIG. 6B, there is binding of rhodamine-labeled APF; in FIG. 6C, there is overlap of images A and B.

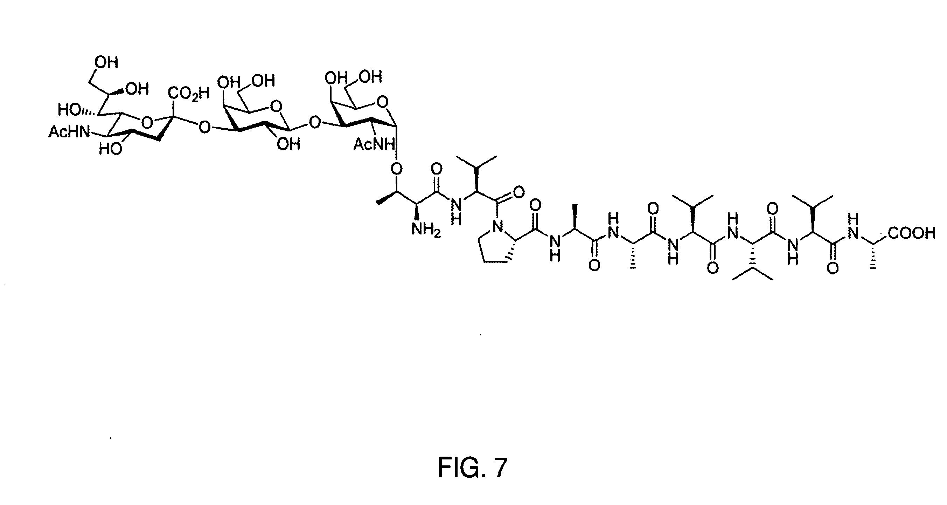

FIG. 7 illustrates an exemplary embodiment of antiproliferative factor from bladder cells.

### DETAILED DESCRIPTION OF THE INVENTION

In keeping with long-standing patent law convention, the words "a" and "an" when used in the present specification in concert with the word comprising, including the claims, denote "one or more." Some embodiments of the invention may consist of or consist essentially of one or more elements, method steps, and/or methods of the invention. It is contemplated that any method or composition described herein can be implemented with respect to any other method or composition described herein.

### I. Definitions

The term "agonist" as used herein refers to a derivative with similar activity to the parent substance, with the same or different potency.

The term "antagonist" as used herein refers to a derivative of the parent substance, or a substance that binds to the parent substance, that can inhibit the activity of the parent substance to varying degrees. The inhibition may be complete or may be partial.

The term "antiproliferative factor" as used herein refers to a molecule comprised of one or more sugar moieties and/or a hydrophobic moiety, wherein the molecule is characterized by the ability to inhibit cell proliferation. In specific embodiments, the inhibiting activity comprises inhibiting epithelial cell proliferation, such as bladder epithelial cell proliferation. In further specific embodiments, the hydrophobic moiety is a peptide or a lipid. In specific embodiments, the hydrophobic nature facilitates nonspecific association with a membrane, or specific or nonspecific interaction with a hydrophobic pocket of a membrane receptor or cytoplasmic receptor, for example. The membrane may be any kind of membrane, although in particular aspects of the invention it is a plasma membrane. In further specific embodiments, the peptide is hydrophobic in part and comprises enough hydrophobicity to facilitate association of APF with a membrane.

The term "APF receptor variant" as used herein refers to a molecule whose structure is derived from the chemical structure of the APF receptor. The variant may be a truncated form of APF receptor, such as one that lacks the C-terminus, the N-terminus, a transmembrane domain, or a combination thereof. There may be conservative substitutions in the variant. In specific cases, the variant comprises no more than 500, 450, 425, 400, 375, 350, 325, 300, 275, 250, 225, 200, 175, 150, 125, 100, 75, 50, or 20 contiguous amino acids of SEQ ID NO:6. In specific cases, the variant comprises at least 500, 450, 425, 400, 375, 350, 325, 300, 275, 250, 225, 200, 175, 150, 125, 100, 75, 50, or 20 contiguous amino acids of SEQ ID NO:6. The variant may be 99%, 97%, 95%, 90%, 85%, 80%, 75%, 70%, or less identical to SEQ ID NO:6. In certain cases, the variant comprises more than one limitation as described in this definition.

The term "bladder disorder" as used herein refers to an abnormal condition of the urinary bladder.

The term "congener" as used herein refers to one or more variants or configurations of a common chemical structure, such as the chemical structure of APF, including the exemplary structure of APF provided in FIG. 7.

The term "urinary bladder" as used herein refers to a distensible membranous sac that serves for the temporary retention of the urine of an individual. Normally it resides in the pelvis in front of the rectum, and it receives the urine from the two ureters, discharging it at intervals into the urethra through an orifice closed by a sphincter. The organ is lined with transitional epithelium.

The term "urinary bladder antiproliferative factor" as used herein refers to an antiproliferative factor as described herein that is associated primarily with the urinary bladder, although in alternative embodiments it is associated with cells from other tissues and/or organs. It may be associated with a cell of the bladder, such as with an epithelial cell, and this then may be referred to as a "urinary bladder epithelial cell antiproliferative factor". The factor may be identified within one or more bladder epithelial cells or it may be identified following secretion from one or more cells, or both. In addition, or alternatively, the factor may be suspended in urine within a bladder or in urine excreted therefrom, or both.

### II. General Embodiments of the Present Invention

The present disclosure generally concerns a receptor for APF and/or its use in identifying agonists or antagonists to APF receptors, APF, or both. The present disclosure generally concerns inhibition of antiproliferative factor (APF) with a particular agent, such as a receptor for APF or a variant thereof.

The receptor may be in any form and from any source, although in particular embodiments it is from bladder epithelial cells, including from one or more membranes of a bladder epithelial cell. The receptor comprises CKAP4/p63. In certain aspects, the receptor is generated synthetically, such as by recombinant DNA.

To isolate potential receptor proteins, the inventors utilized an exemplary method of solubilizing microsomal preparations from APF-sensitive bladder epithelial cells that were passed over an avidin column to which biotinylated, synthetic APF was attached. After a series of high stringency washing steps, two proteins were observed in the eluate and identified by mass spectrometry as cytoskeletal-associated protein 4 (CKAP4/p63) and vimentin. Demonstration that CKAP4/p63 is a functional receptor for APF was accomplished in an exemplary manner through the use of anti-CKAP4/p63 antibodies that effectively inhibited APF activity. Additional evidence was provided by decreased normal bladder epithelial cell sensitivity to APF following siRNA knockdown of CKAP4/p63. Co-localization of CKAP4/p63 and APF-binding in bladder epithelial cells was confirmed by confocal immunofluorescence microscopy.

### III. Receptor for Antiproliferative Factor (APF)

The present invention relates to a receptor for antiproliferative factor (APF), such as an antiproliferative factor from bladder epithelial cells, which is CKAP4/p63. The receptor may endogenously reside in a membrane of at least one type of cell, including an epithelial cell, such as, for example, a bladder cell, although in particular embodiments, a soluble form of the receptor is employed. In particular, one or more transmembrane domains or other domain(s) may be removed from the receptor.

As its receptor, APF may directly or indirectly bind CKAP4/p63. An exemplary CKAP4/p63 polypeptide is provided in SEQ ID NO:6 (National Center for Biotechnology Information GenBank® database's GenBank® Accession number AAH94824). An exemplary CKAP4/p63 polynucleotide is provided in SEQ ID NO:7 (GenBank® Accession number BC082972). Another exemplary CKAP4 polypeptide is provided in SEQ ID NO:11 (GenBank® Accession number NP_006816) and another exemplary CKAP4 polynucleotide is provided in SEQ ID NO:12 (GenBank® Accession number NM_006825). An exemplary vimentin polypeptide is provided in SEQ ID NO:8 (GenBank® Accession number NP_003371). An exemplary vimentin polynucleotide is provided in SEQ ID NO:9 (GenBank® Accession number M14144).

In some aspects, the receptor is employed as a nucleic acid that encodes at least part of the receptor, although in other embodiments the receptor is employed as a polypeptide. In either case, the receptor may comprise the part of the receptor that is extracellular and soluble.

In some embodiments, the APF receptor is further characterized, such as by x-ray crystallography, NMR studies, far western, or a combination thereof. The APF receptor may be further characterized in whole, in one or more parts, or both, and at least part of the receptor, including, for example, the APF binding site, may be employed for identification of agonists and/or antagonists of APF and/or the APF receptor.

### IV. Exemplary Isolation Scheme for Receptor of APF

The following describes at least one method of isolating a receptor for APF, although one of skill in the art is aware of other means to do so, such as crosslinking of APF to cell membrane proteins, for example. An exemplary APF biotinylated on the C-terminal alanine residue was immobilized on a streptavidin chromatographic solid phase support. Bladder epithelial cells that were explanted from bladder epithelial cells of normal individuals and shown to be sensitive to APF's antiproliferative effects were grown from low passage number stocks (<
6 passages) to confluence in T150 tissue culture flasks containing MEM with 10% fetal calf serum, 1% L-glutamine, and 1% antibiotic/antimycotic solution (all from Sigma). Cell membrane proteins were solubilized in Tris buffered saline containing Triton X-100 and protease inhibitors and loaded onto an APF-affinity spin column generated by immobilizing a C-terminal biotinylated synthetic version of APF onto a 100 streptavidin immobilized on agarose bead stationary phase support (100 mL bed volume, Pierce Biotechnology, Rockford, IL). The column was washed four times sequentially with 500 mL each of 50, 100, 250 500 mM NaCl in 100 mM NH₄HCO₃, pH 7.9, and finally with 1 M NaCl in 100 mM NH₄HCO₃, pH 7.9. Each of the recovered washes was lyophilized, resuspended in Laemli buffer and resolved by one dimensional (1D) sodium dodecyl sulfate (SDS) polyacrylamide gel electrophoresis (PAGE) followed by visualization with silver stain. In addition, a corresponding control experiment was performed with no biotinylated APF immobilized to the streptavidin-agarose stationary phase (to evaluate non-specific binding to the stationary phase).

Two protein bands were visualized that were uniquely isolated by the APF-affinity column (as compared to the control column). These proteins were then excised, digested from the gel by trypsin, and the extracted tryptic peptides analyzed by nanoflow reversed-phase liquid chromatography (nanoRPLC) coupled online with tandem mass spectrometry (MS/MS). The peptides (hence protein) were identified by searching the tandem MS data against the non-redundant human proteome database, and found to be homologous to CAPK4/p63 and vimentin. In some aspects of the invention, this protein can be further characterized by Western blot, as well as its selective binding to active APF further characterized, such as by Far-Western blot, for example.

In certain aspects, an APF receptor is isolated from the nucleus, such as from the nuclear membrane or from the nuclear cytosol, such as the nuclear membrane or nuclear cytosol of an epithelial cell, including a bladder epithelial cell. The isolation of APF receptor from the nucleus may comprise fractionating cell components, such as nuclear from non-nuclear, and isolating the receptor from the nuclear fractions.

In other aspects, it is determined if APF receptor is associated with DNA, either directly or indirectly. In particular, a gel shift assay may be utilized to determine if APF receptor binds DNA. Exemplary DNAs to employ include sequences from regulatory regions of polynucleotides known to be regulated by certain transcription factors that regulate genes associated with a change in expression for bladder epithelial cell expansion (Keay et al., 2003; Physiol. Genomics) In other embodiments, APF may bind the transcription factor.

### V. Antiproliferative Factor (APF)

APF comprises a glycopeptide that at least inhibits proliferation of bladder epithelial cells, skin fibroblasts, and other epithelial cells including prostate cells, and in some embodiments is generated by bladder epithelial cells, such as those associated with interstitial cystitis. In particular embodiments, the compound is present in the urine of individuals having interstitial cystitis. In other embodiments, the compound is generated or biosynthesized by tissues and cells other than urinary bladder tissue and cells. In one aspect the compound is considered a toxin, a negative growth factor, or both.

APF was identified because of its ability to inhibit the growth of cells that line the bladder wall, in specific embodiments by altering the production of several proteins by these cells, such as specific growth factors and cell adhesion proteins. Not to be bound to any theory, in further embodiments APF causes interstitial cystitis in which the bladder lining is generally thin and/or ulcerated.

Thus, as used herein the term "APF" refers to a class of compounds wherein the structure in FIG. 7 is merely the prototypical APF and other related compositions are encompassed as a ligand for the receptor of the invention. Although in particular aspects APF comprises the structure provided in FIG. 7, this is merely one embodiment of a ligand for the receptor. A skilled artisan recognizes that the structure in FIG. 7 may bind a receptor, although in some embodiments a similar but non-identical structure of APF binds the receptor; the binding of the similar but non-identical structure of APF may be in addition to or instead of the binding of the structure in FIG. 7 to the receptor. APF compositions that bind the receptor herein encompass both isolated natural APF, synthetic versions thereof, derivatives thereof, or a mixture thereof. Furthermore, the APF receptor compounds may be derived using synthetic means or isolated from a natural source, such as bladder epithelial cells, their extracellular medium, tissue or bodily fluids such as urine, serum, or plasma, for example.

Furthermore, inhibition of a molecule having the structure of APF in FIG. 7 by a receptor is useful, but inhibition of a molecule having a similar structure of APF may also be useful. The inhibition with the receptor may be directed to inhibiting the function of APF.

Thus, in specific embodiments, compositions related to the APF that is capable of associating with the APF receptor comprise about one to about six sugar residues; and a peptide of about two to about fifteen amino acid residues, wherein the peptide-linked to one of the sugar moieties at a linking amino acid, wherein the linking amino acid comprises a heteroatom which serves as the linking portion of the linking amino acid. More specifically, the linking amino acid comprises a serine, a threonine, or a cysteine. In other specific embodiments, the compositions of the present invention comprises two or three sugar residues and nine amino acids and the linking amino acid is a threonine or serine.

In one specific aspect, APF is an acidic, heat stable sialoglycopeptide comprising 9 amino acid residues (such as, for example, TVPAAVVVA, SEQ ID NO:1; SVPAAVVVA, SEQ ID NO:2; TVPAAVVLA, SEQ ID NO:3; or SLPAAVVVA, SEQ ID NO:4) covalently linked through the N-terminal threonine, serine, or cysteine, for example, to an N-acetylgalactosamine or N-acetylglucosamine residue that is linked *via* an α- or β- configuration to galactose, and sialylated on the galactose moiety *via* 2,3 linkage. The anomeric configuration of the glycosyl bond is alpha in particular embodiments, although it may be beta in alternative embodiments.

In one particular aspect, an APF composition may comprise in part a hydrophobic moiety, such as a peptide, for example one including SEQ ID NO:1, SEQ ID NO:2, or SEQ ID NO:3, SEQ ID NO:4, or a lipid. The peptide may comprise at least part of a transmembrane domain, and in particular embodiments it comprises part of frizzled 8, such as a transmembrane domain of frizzled 8. In specific embodiments, the peptide is hydrophobic.

The glycoprotein comprising a galactose covalently linked to an N-acetylglucosamine or an N-acetylgalactosamine covalently linked to a peptide of SEQ ID NO: 1 or variants thereof is provided herein. The term "variants thereof," in the context of APF, includes peptidomimetics of various types (Ahn *et al.,* 2002). The peptides may comprise any suitable amino acids, such as L-amino acids, D-amino acids, N-methylated amino acids, or a combination thereof, as well as peptidomimetic compounds such as unnatural amino acids or other "peptide-like" organic constructs that mimic the specific structural elements of a linear, cyclic, or branched peptide that correspond to active peptides. The sugar moieties may be natural, synthetic, carbohydratemimetic, or a mixture thereof may be used in a composition. Glycopeptidomimetic compounds where the sugars are carbohydratemimetic moieties or the peptide components are peptidomimetic moieties, or a combination of the two, are encompassed in the invention. In specific embodiments, the sugars of the present invention include amino sugars.

In a particular aspect, the APF has a molecular mass of 1482.8 and comprises nine amino acids and three sugar moieties in the following order: (a) Sialic acid-galactose-N-acetylgalactosamine-threonine-valine-proline-alanine-alanine-valine-valine-valine-alanine; or (b) Sialic acid-galactose-Nacetylglucosamine-threonine-valine-proline-alanine-alanine-valine-valine-valine-alanine; or (c) Sialic acid-galactose-N-acetylglucosamine-serine-leucine-proline-alanine-alanine-valine-valine-valine-alanine. The composition may be further defined as having one or more of the following: the sialic acid in (a) is linked to galactose *via* a 2,3 linkage; the sialic acid in (b) is linked to galactose *via* a 2,3 linkage; the sialic acid in (c) is linked to galactose *via* a 2,3 linkage; the galactose in (a) is linked to the N-acetylgalactosamine *via* a 1,3 linkage; the galactose in (b) is linked to the N-acetylglucosamine *via* a 1,4 linkage; the galactose in (c) is linked to the N-acetylglucosamine *via* a 1,4 linkage; the N-acetylglucosamine is linked to serine *via* an O linkage in an alpha configuration; or the N-acetylgalactosamine is linked to threonine or serine *via* an O linkage in an alpha configuration.

It is contemplated that APF compositions may be modified so as to improve certain characteristics, such as solubility by adding a water soluble unit. The term "water soluble unit" means any functional group imparting water solubility, including, but not limited to, SO₃-, PO₃²⁻, CH₂, COO-, a quaternary ammonium group attached *via* an ester or alkyl linkage such as C=O(CH₂)x NAlk₃ or (CH₂)x NAlk₃ where Alk₃ represents three alkyl groups that are independently C1-C4 alkyl and x is 1-4, (CH₂ CH₂ O)n CH₂ CH₂ OX (n=1-3) wherein X may be H or CH₃, *i.e.,* PEG or MeO-PEG. The counterion for water soluble units bearing a charge include, but are not limited to, metals such as alkali and alkaline earth metals, and halogens.

Certain APF compounds comprise a threonine, a serine, or a cysteine at the N- terminus or any functional equivalent. Non-limiting examples of functional equivalents include a synthetic derivative having a primary or secondary or tertiary alcohol, an ester, a carboxylic acid, an ether, a thiol, a thiolate, or any functional group enabling for covalent linkage with a sugar molecule, provided the molecule retains biological function.

Other functionalities contemplated in "derivatives" include isomers of any of the sugars or amino acids, whether positional, structural, or stereoisomers. Other substituents known to those skilled in the chemical arts may be provided, so long as the biological function of the molecule is retained, in specific embodiments of the invention.

### VI. Interference or Enhancement of APF Interaction with APF Receptor

In some embodiments one or more agents that interfere with the binding of APF to its receptor are identified, and in specific embodiments, the one or more agents are employed therapeutically for an individual in need thereof, such as an individual that has or is susceptible to interstitial cystitis. Such an inhibitor agent may be referred to as an antagonist, which is defined as one or more molecules that interfere with the activity of a different molecule, including interfering with the binding of the molecule to its receptor or ligand, for example. In other embodiments one or more agents that improve or enhance the interaction of APF with its receptor are identified, and in specific embodiments, the one or more agents are employed therapeutically for an individual in need thereof, such as an individual that has or is susceptible to hyperproliferative disease, including bladder or kidney cancer, for example. Such an enhancing agent may be referred to as an enhancer, which may be defined as one or more molecules that enhance the activity between two other molecules. In other embodiments for treatment of hyperproliferative disorder, there are agents that are identified that enhance production or activity of APF receptor or that increase expression of receptor in a cell such that it renders the cell susceptible to APF.

### A. Agents that Interfere with APF Interaction with APF Receptor

In specific embodiments, an agent is identified or obtained that interferes at least in part with the interaction of APF with its receptor (or with any APF activity).

### 1. APF Analog

The agent may be an APF analog, in some embodiments, although in other embodiments the agent is not an APF analog. An APF analog may be defined as an APF receptor-binding congener of APF (and these terms may be used interchangeably). In examples where the agent is an APF analog, the modifications of the analog compared to APF may be in the sugar portion, the peptide portion, or both, for example. Such modifications may affect the activity of the APF analog, such as reduce APF activity at least in part. In some aspects , the peptide portion of APF is altered, such as one or more amino acids being removed and/or replaced. For example, one or more amino acids may be substituted with another amino acid. In certain but exemplary aspects, the peptide comprises TVPAAVVA (SEQ ID NO:10), and in further aspects one or more of the amino acids of SEQ ID NO: 10 is altered or removed. In specific embodiments, one or more amino acids are truncated, and in particular embodiments two or more amino acids are truncated from the C-terminus of the peptide. In particular embodiments, a proline in the peptide is modified, such as to reduce the extent of a bend in the peptide or to increase the extent of a bend in the peptide. For example, an amino acid substitute having a four-membered ring, which in some embodiments enforces a bend more severe than that imparted by proline, renders the APF analog as being inactive.

In other aspects, one or more sugar moieties of APF are modified and/or removed. In particular aspects, one, two, or all three sugar residues are removed. In certain aspects, two sugars are removed. In specific embodiments, the second sugar residue of APF is β-galactose, and this sugar is altered.

In other embodiments of agents that interfere with APF binding to APF receptor, a small molecule may be identified. For example, a library of small molecules may be employed in an assay to identify agents that render APF activity and/or binding to its receptor reduced or undetectable, such as abolished. The assay may be a high throughput assay, for example.

### 2. APF Receptor Derivative

Agents that interfere with APF activity, such as binding to APF receptor, may be identified at least in part by characterizing the structure of APF, APF receptor, or both. An APF receptor derivative may be further defined as an analog of CKAP4, which may also be referred to as a variant of CKAP4. The APF receptor analog may comprise one or more domains of CKAP4, although in certain aspects the APF receptor analog comprises at least part of the domain that binds APF.

In certain aspects, the APF receptor analog comprises a variant of CKAP4, which in specific embodiments may be referred to as a polypeptide of SEQ ID NO:6 that comprises at least one amino acid difference with SEQ ID NO:6. The derivative may lack one or more transmembrane domains, may be soluble, and so forth.

### 3. Identification of Agents that Interfere with APF or APF Receptor

The structure of APF, CKAP4 (APF receptor), or both may be observed to identify one or more regions suitable for producing molecules that mimic their structure and thus are useful for inhibiting or enhancing binding of APF to APF receptor. Such studies may comprise x-ray crystallography, NMR, or other examples suitable in the art. The APF or APF receptor may be observed in such studies in whole or in part.

In a specific embodiment, a high throughput assay for APF agonists and antagonists is employed. The finding that the cellular receptor for APF is the cytoskeleton associated protein 4 (CKAP4) allows one to employ high throughput screens for antagonists and agonists of APF. In specific embodiments, CKAP4 is a membrane protein that is targeted by at least one other agonist, namely the tissue plasminogen activator protein (tPA). Although in some embodiments the entire CKAP4 protein is utilized as a target for HTS, in other embodiments a truncated form of the protein comprising the extracellular domain of CKAP4 and at least part of the transmembrane region is used as the substrate for the assay. In specific embodiments of the invention, APF and/or tPA bind to the extracellular domain. This or any other fragment of CKAP4 may be cloned and expressed. Two types of exemplary assays are proposed for any compositions of the invention.

1. For antagonist screening, a highly automated version of an ELISA assay may be used. APF that is labeled on the C-terminus, for example, with a label such as a rhodamine dye, for example, is premixed in a multi-well plate with the exemplary CKAP4 molecule, such as the CKAP4 fragment referred to above. This is followed by addition of individual components of a chemical library, each added at different concentrations, such as three different concentrations, for example. Loss of dye fluorescence is indicative of antagonism, in certain aspects of the invention. An exemplary library to employ is the highly diverse Cambridge library (~300K compounds) for the initial screen.

2. Agonist screening may be accomplished by attaching the CKAP4 fragment to the gold surface of a surface plasmon resonance spectrometer cell (a Biacore instrument may be employed). The putative agonist(s) is flowed through the instrument, and binding curves are determined. The data is quantitative and provides a firm measure of the strength of binding of potential agonist(s) to the protein. This is compared to binding curves obtained for APF itself, for example.

### B. Agents that Enhance APF Interaction with APF Receptor

In particular embodiments of the invention, there are one or more agents that enhance APF interaction with an APF receptor.

The agent may be an APF analog, in some embodiments, although in other embodiments the agent is not an APF analog. In examples where the agent is an APF analog, the modifications of the analog compared to APF may be in the sugar portion, the peptide portion, or both, for example. Such modifications may affect the activity of the APF analog, such as increase any APF activity, including its binding to an APF receptor.

In some aspects, the peptide portion of APF is altered, such as one or more amino acids being removed and/or replaced. For example, one or more amino acids may be substituted with another amino acid. In certain aspects, the peptide comprises a proline in the peptide that is modified, such as to reduce the extent of a bend in the peptide or to increase the extent of a bend in the peptide. For example, an amino acid substitute having a six-membered ring, which in some embodiments reduces the bend imparted by proline, renders the APF analog as being an active agonist.

In other aspects, one or more sugar moieties of APF are modified and/or removed or added.

In other embodiments of agents that enhance APF binding to APF receptor, a small molecule may be identified. For example, a library of small molecules may be employed in an assay to identify agents that render APF activity and/or enhanced binding to its receptor. The assay may be a high throughput assay, for example.

### VII. Exemplary Assays to Identify Agents that Interfere with APF Binding to APF Receptor

In particular embodiments, there is an assay that identifies an agent that interferes with binding of APF to the APF receptor. In specific embodiments, the assay comprises providing APF, one or more test agents, and the APF receptor, and determine whether there is binding of APF to the APF receptor. In specific cases, there is a method of screening for an inhibitor of APF binding to the APF receptor, comprising: (a) providing a candidate inhibitor; (b) admixing the candidate inhibitor with APF, APF receptor-binding congener of APF, APF receptor, and/or APF receptor derivative; (c) measuring binding of APF or the APF receptor-binding congener to the APF receptor or the APF receptor derivative; and (d) comparing whether there is binding in step (c) with the binding of a control, said control optionally comprising APF or the APF receptor-binding congener binding to the APF receptor or the APF receptor derivative in the absence of the candidate inhibitor, wherein a difference in binding between the candidate inhibitor embodiment and the control embodiment indicates said candidate inhibitor is an inhibitor of binding of APF to the APF receptor.

In another embodiment, there is an assay for antagonistic activity against APF that may be performed the same or similar to an exemplary ³H-thymidine incorporation assay or other cell proliferation assay known to those of skill in the art using primary normal bladder epithelial cells with the following single exception: following overnight serum-starvation, the cells are incubated with varying concentrations of the potential antagonist diluted in phosphate buffered saline for 1.5 hours at 37°C before APF is added to the cell medium. Similarly, cells or purified receptor could be preincubated with an antagonist prior to APF, and APF activity or binding of labeled APF is detected. Other assays for cell proliferation include live cell counts, BrdU incorporation, MTT assay, or other assays that measure nucleic acid incorporation, increase in cell number, increase in cell respiration, or other indications of cell growth.

### VIII. Exemplary Assays to Identify Agents that Enhance APF Binding to APF Receptor

An assay to identify agents that enhance APF, APF receptor, or binding between the APF and APF receptor is utilized. Assays for APF binding to its receptor include far western assays, affinity purification, binding of labeled APF (radiolabeled, biotinylated, or other, for example) or detection of APF binding by immunoassay using antibodies against APF and/or its receptor.

### IX. Exemplary Assays to Identify Agents that Inhibit Cell Proliferation

In certain embodiments of the invention, one or more assays may be employed to identify one or more agents that inhibit cell proliferation, and in certain aspects the agent is useful for cancer therapy. In other certain embodiments, an anti-cell proliferation agent is identified by utilizing an APF receptor or variant thereof, for example by identifying one or more compounds that binds APF receptor. Binding of APF receptor with one or more candidate compounds may be performed by any suitable method known in the art.

In one exemplary assay, a compound suspected of being an anti-cell proliferation agent (which may be considered a candidate compound and may be an APF variant, an APF receptor variant, or identified by its interaction with an APF variant or an APF receptor variant) is provided to a cell, and the growth, such as growth rate, following said providing is monitored. When the growth, such as growth rate, is reduced compared to in the absence of the compound, then the compound suspected of being an anti-cell proliferation agent is a anti-cell proliferation agent. In other aspects of the invention, a compound suspected of being an anti-cell proliferation agent is delivered to an animal model, such as a mouse cancer model (including a mouse bladder cancer model or a mouse kidney cancer model), and the growth of the cancer in the mouse, such as being monitored by tumor volume, for example, is monitored. When the cancer in the mouse is inhibited at least partially, then the compound suspected of being an anti-cell proliferation agent is an anti-cell proliferation agent. A high throughput system may be utilized to identify the anti-cell proliferation agent.

### X. Therapeutic and/or Preventative Embodiments

The APF receptor may be employed to identify one or more agents that are utilized for therapeutic and/or preventative purposes. For example, in therapeutic embodiments wherein proliferation is desirable, such as with interstitial cystitis, an inhibitor of APF activity or production, an inhibitor of APF binding to another molecule, such as its receptor, and/or a stimulator of APF breakdown, may be employed for therapy.

In one aspect, a deleterious bladder condition is associated either indirectly or directly with reduced cellular proliferation, such as inhibited epithelial cellular proliferation. Such a condition may result in harmful alterations to the bladder epithelium such that it would be beneficial to reduce or substantially remove the inhibition of cellular proliferation. In these aspects, it is desirable to deliver to the individual with the bladder condition, such as deliver systemically to the individual or directly to bladder epithelium, for example, a composition that at least improves at least one symptom of the condition. Such a composition may comprise an inhibitor of an APF, an inhibitor of APF receptor, or an inhibitor of binding of APF to the APF receptor, such as may be identified in an assay as described herein, for example. The composition may be delivered by any suitable means, although in specific embodiments it is delivered *via* catheter, systemically, orally, intravenously, topically, subcutaneously, transcutaneously, intramuscularly, intra-jointly, parenterally, peritoneally, intranasally, intravesically or by inhalation. In other specific embodiments, the composition is comprised in a pharmaceutically acceptable excipient, such as an aqueous or non-aqueous liquid. In particular aspects, it is administered in a non-aqueous excipient due to the hydrophobic nature of the peptide moiety. It may be delivered alone or in a carrier, such as a liposome, encapsulated cell, viral vector, nanoparticles, biodegradable gel or polymer, implanted osmotic pump, or other suitable devices.

In another aspect, a deleterious bladder condition is associated either indirectly or directly with increased cellular proliferation, such as increased epithelial cellular proliferation. Such a condition may result in malignancy of the bladder epithelium, such that it would be beneficial to reduce in part or substantially in full the amount of cellular proliferation. In these aspects, it is desirable to deliver to the individual with the bladder condition, a compound that enhances production or activity of APF receptor and/or an expression construct that produces the APF receptor to make the cell more sensitive to APF. The delivery of the APF composition may be systemically to the individual or directly to bladder epithelium. The composition may be delivered by any suitable means, although in specific embodiments it is delivered *via* catheter, orally, intravenously, topically, subcutaneously, transcutaneously, intramuscularly, orally, intra-jointly, parenterally, peritoneally, intranasally, intravesically or by inhalation. In other specific embodiments, the composition is comprised in a pharmaceutically acceptable excipient. It may be delivered alone or in a carrier, such as a liposome, encapsulated cell, vector, (including a non-viral vector or a viral vector, for example an adenoviral vector, retroviral vector, adeno-associated viral vector, and so forth), nanoparticles, biodegradable gel or polymer, implanted osmotic pump, or other suitable devices.

In a particular embodiment, a composition may be administered to an individual with any kind of cancer, including epithelial cancers. In specific embodiments, there is a malignancy of the bladder epithelium, which may be referred to herein as bladder cancer. In specific embodiments, there is a cancer therapy additional to the present inventive treatment, such as gene therapy, chemotherapy, radiation, surgery, immunotherapy, or a combination thereof. In other embodiments, kidney cancer is treated and/or prevented with one or more compositions of the invention.

### XI. Bladder Disorders

Although the present disclosure may be useful for any medical condition for which an APF receptor, APF receptor agonist, or APF receptor antagonist provides therapy and to any individual in need thereof, specific embodiments apply to one or more bladder disorders. Although the terms "bladder disorder" or "bladder condition" refer to any abnormal condition of the urinary bladder, in specific embodiments the bladder disorder comprises interstitial cystitis, bladder cancer, either as a primary or secondary cancer, chronic pelvic pain syndrome, irritable bladder syndrome, urethral syndrome, painful bladder syndrome, chronic nonbacterial prostatitis, and other bladder conditions characterized by increased urinary frequency often accompanied by bladder pain and/or increased urinary urgency, for example, and in some cases for which no other etiology has been determined.

In specific embodiments, there are methods and compositions related to interstitial cystitis. Typical symptoms of interstitial cystitis include pain, which can be in the abdominal, urethral or vaginal area and is also frequently associated with sexual intercourse; urgency, which includes the sensation of having to urinate immediately and may also be accompanied by pressure and/or spasms; and increased frequency of urination, which can be day and/or night frequency of urination.

Diagnosis of intersitial cystitis is heretofore performed using cystoscopy, and hydro-distention and biopsies are normally performed at the same time. Examination by cytoscopy of a typical bladder having interstitial cystitis may identify submucosal pinpoint hemorrhages (glomerulations), thinning of the epithelium and/or Hunner's ulcers; in some cases, inflammation may also be present. Thus, there is considerable pain when urine enters into the bladder of an IC patient, making it very difficult for patients with interstitial cystitis to be able to hold urine in their bladder, due to the burning, stinging and pain.

Current therapies include oral medications, such as Elmiron®, Amitriptyline (Elavil®) Atarax®, Neurontin®, Ditropan®, Prozac®, and Cimetidine, for example. Therapies include pentosan polysulfate, amitriptyline, hydroxyzine, gabapentin, oxybutynin, fluoxetine, cimetidine, cyclosporin A, dimethyl sulfoxide, neurostimulation, hydrodistention, Bacille Calmette-Guérin, or mycophenolate mofetil. Therapeutic agents may be used either alone or in conjunction with one or more of these or similar medications. In specific embodiments, the patients also suffer with various other syndromes including fibromyalgia, urethral syndrome, vulvodynia, irritable bowel syndrome, chronic fatigue syndrome, allergies, and other autoimmune disorders, such as scleroderma, systemic lupus erythematous, for example, that may be associated with interstitial cystitis.

### XII. Pharmaceutical Compositions

In particular embodiments, there are pharmaceutical compositions for use in treating and/or preventing bladder conditions, such as interstitial cystitis, for example. It is further contemplated that the compounds may be used to block the interaction of APF with its target for the treatment of interstitial cystitis or other disorders related to cell proliferation. In particular, the APF receptor that is employed in such a method may be soluble in form, and upon binding of APF to this soluble form, the APF is prevented from binding at least one of its natural targets, such as APF receptor that resides in one or more membranes. Thus, compounds that inhibit the interaction of APF with the receptor are utilized for treatment of interstitial cystitis.

In other embodiments, there are pharmaceutical compositions for treating or ameliorating hyperproliferative disorders, such as bladder cancer, epithelial hyperplasia or malignancies of epithelial origin, of fibroblast hyperplasia or malignancy, other solid tumors, or lymphoreticular malignancies, for example. Such compounds are also contemplated for use as an adjuvant treatment for bladder cancer or other malignancies, as an antiangiogenic agent or an antifungal agent. The hyperproliferative disease compounds will enhance APF binding to the APF receptor, in specific cases. In certain aspects, there is en expression construct that encodes APF receptor, or a fragment thereof, and in further aspects, such expression renders the cell more sensitive to APF. In further specific embodiments, these cells have overexpression of the APF receptor. In other certain embodiments, administration of the composition for treating hyperproliferative disorders includes local administration if the hyperproliferation is localized or systemic if the hyperproliferation is not localized.

Embodiments generally involve administering a pharmaceutical composition comprising an effective amount of the composition as described above. Where treating with compounds , administration of the compounds with a suitable pharmaceutical excipient as necessary can be carried out *via* any of the accepted modes of administration. The compounds may be comprised in a pharmaceutically acceptable excipient, which may be considered as a molecular entity and/or composition that does not produce an adverse, allergic and/or other untoward reaction when administered to an animal, as appropriate. It includes any and/or all solvents, dispersion media, coatings, antibacterial and/or antifungal agents, isotonic and/or absorption delaying agents and/or the like. The use of such media and/or agents for pharmaceutical active substances is well known in the art. Except insofar as any conventional media and/or agent is incompatible with the active ingredient, its use in the therapeutic compositions is contemplated.

Thus, administration can be, for example, systemicintravenous, topical, subcutaneous, transcutaneous, intramuscular, oral, intra-joint, parenteral, peritoneal, intranasal, intravesical or by inhalation. Suitable sites of administration thus include, but are not limited to, skin, bronchial, gastrointestinal, anal, vaginal, eye, bladder, and ear. The formulations may take the form of solid, semi-solid, lyophilized powder, or liquid dosage forms, such as, for example, tablets, pills, capsules, powders, solutions, suspensions, emulsions, suppositories, retention enemas, creams, ointments, lotions, aerosols or the like, preferably in unit dosage forms suitable for simple administration of precise dosages.

The compositions typically include a conventional pharmaceutical carrier or excipient and may additionally include other medicinal agents, carriers, adjuvants, and the like. Preferably, the composition will be about 5% to 75% by weight of a compound or compounds of the invention, with the remainder consisting of suitable pharmaceutical excipients. Appropriate excipients can be tailored to the particular composition and route of administration by methods well known in the art, *e.g*., REMINGTON'S PHARMACEUTICAL SCIENCES, 18TH ED., Mack Publishing Co., Easton, Pa. (1990).

The compositions may be administered to the bladder directly, such as by catheter, or it may be released as by an osmotic pump. It may also be made directly by bladder cells that have been transfected with nucleic acid or a viral agent, for example, carrying nucleic acid that encodes the receptor in a soluble form that is then secreted by the cell or in a membrane-bound form for expression at the cell membrane.

For oral administration, such excipients include pharmaceutical grades of mannitol, lactose, starch, magnesium stearate, sodium saccharine, talcum, cellulose, glucose, gelatin, sucrose, magnesium carbonate, and the like. The composition may take the form of a solution, suspension, tablet, pill, capsule, powder, sustained-release formulation, and the like.

In some embodiments, the pharmaceutical compositions take the form of a pill, tablet or capsule, and thus, the composition can contain, along with the biologically active conjugate, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof.

The active compounds of the formulas may be formulated into a suppository comprising, for example, about 0.5% to about 50% of a compound of the invention, disposed in a polyethylene glycol (PEG) carrier (*e.g*., PEG 1000 [96%] and PEG 4000 [4%]).

Liquid compositions can be prepared by dissolving or dispersing compound (about 0.5% to about 20%), and optional pharmaceutical adjuvants in a carrier, such as, for example, aqueous saline (*e.g*., 0.9% w/v sodium chloride), aqueous dextrose, glycerol, ethanol and the like, to form a solution or suspension, *e.g*., for intravenous administration. The active compounds may also be formulated into a retention enema.

If desired, the composition to be administered may also contain minor amounts of non-toxic auxiliary substances such as wetting or emulsifying agents, pH buffering agents, such as, for example, sodium acetate, sorbitan monolaurate, or triethanolamine oleate.

For topical administration, the composition is administered in any suitable format, such as a lotion or a transdermal patch. For delivery by inhalation, the composition can be delivered as a dry powder (*e.g.,* Inhale Therapeutics) or in liquid form *via* a nebulizer.

Methods for preparing such dosage forms are known or will be apparent to those skilled in the art; for example, see Remington's Pharmaceutical Sciences, supra., and similar publications. The composition to be administered will, in any event, contain a quantity of the pro-drug and/or active compound(s) in a pharmaceutically effective amount for relief of the condition being treated.

Generally, the compounds are administered in a therapeutically effective amount, *i.e*., a dosage sufficient to effect treatment, which will vary depending on the individual and condition being treated. Typically, a therapeutically effective daily dose is from 0.1 to 100 mg/kg of body weight per day of drug. Most conditions respond to administration of a total dosage of between about 1 and about 30 mg/kg of body weight per day, or between about 70 mg and 2100 mg per day for a 70 kg person. However, it is possible that an effective dose of APF, especially if administered directly into the bladder, may be outside of this range.

Stability of the conjugate can be further controlled by chemical alterations, including D amino acid residues in the polypeptide chain as well as other peptidomimetic moieties. Furthermore, stability of the conjugates could also be enhanced by unnatural carbohydrate residues.

### XIII. Combination Treatments

In particular aspects, the compounds are are employed in combination with one or more other therapies. In certain embodiments, the therapy is for a bladder disorder, including, for example, bladder cancer, interstitial cystitis, and so forth. In other embodiments, the other therapy is for cancer.

Treatment may precede, follow, or both precede and follow the other treatment by intervals ranging from minutes to weeks. In embodiments where the composition and the other agent are applied separately to a cell of the individual, such as *via* the luminal side of the bladder, one would generally ensure that a significant period of time did not expire between the time of each delivery, such that the APF receptor composition and the other treatment would still be able to exert an advantageously combined effect on at least one cell associated with the bladder disorder. In such instances, it is contemplated that one may instill both modalities within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

Various combinations may be employed, for.example, wherein the present inventive treatment is "A" and the secondary agent, such as the respective bladder disorder or cancer treatment, is "B":

A/B/A B/A/B B/B/A A/A/B A/B/B B/A/A A/B/B/B B/A/B/B

B/B/B/A B/B/A/B A/A/BB A/B/AB A/B/B/A B/B/A/A

B/A/B/A B/A/AB A/A/AB B/A/A/A AB/A/A A/AB/A

Administration of the APF or APF receptor compositions to a patient will follow general protocols for the administration of chemotherapeutics, taking into account the toxicity, if any, of the molecule. It is expected that the treatment cycles would be repeated as necessary. It also is contemplated that various standard therapies, as well as surgical intervention, may be applied in combination with the described hyperproliferative cell therapy.

### A. Interstitial Cystitis Treatment Combinations

In one aspect APF/APF receptor-binding inhibitors compositions are employed in combination with one or more therapies for interstitial cystitis (IC), and in particular aspects the combination with other IC therapies increases the effectiveness of the APF receptor composition, increases the treatment that it is combined with, or both. The combination of the therapy with another IC therapy may provide additive therapeutic effects or synergistic therapeutic effects, for example. Exemplary IC treatment includes Elmiron®, Amitriptyline (Elavil@) Atarax®, Neurontin@, Ditropan®, Prozac®, Cimetidine, and combinations thereof, for example. The treatment of interstitial cystitis may comprise pentosan polysulfate, amitriptyline, hydroxyzine, gabapentin, oxybutynin, fluoxetine, cimetidine, cyclosporin A, dimethyl sulfoxide, neurostimulation, hydrodistention, Bacille Calmette-Guérin, mycophenolate mofetil, or a combination or mixture thereof.

### B. Cancer Treatment Combinations

In certain aspects, there is treatment of cancer employing a compound that enhances production or activity of the APF receptor and/or employing a compound that comprises an expression construct that encodes APF receptor or a fragment thereof. In some cases, such APF receptor-related embodiments are further employed with another cancer treatment, such as chemotherapy, radiation, surgery, and so forth. In particular aspects the combination with other cancer therapies increases the effectiveness of the cancer therapy. The combination of the therapy disclosed herein with another cancer therapy may provide additive therapeutic effects or synergistic therapeutic effects.

In one aspect of the invention, there is identification of agents that could be employed in combination with one or more therapies for cancer, such as bladder cancer, such as by employing an APF receptor composition to identify agents that bind to it and, in further embodiments, have cell inhibition activity.

In order to increase the effectiveness of an APF receptor, APF receptor agonist, or APF receptor antagonist composition for the treatment of cancer in an individual, such as a patient, it may be desirable to combine these compositions with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy, for example, by combining it with other cancer therapies. In is contemplated that APF receptor, APF receptor agonist, or APF receptor antagonist composition therapy could be used similarly in conjunction with chemotherapeutic, radiotherapeutic, surgical, or immunotherapeutic intervention, in addition to other pro-apoptotic or cell cycle regulating agents.

### 1. Chemotherapy

A skilled artisan recognizes that in addition to the appropriate compositions described herein for the purpose of inhibiting cell growth, other chemotherapeutic agents are useful in the treatment of neoplastic disease. Examples of such chemotherapeutic agents include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein transferase inhibitors, transplatinum, 5-fluorouracil, vincristin, vinblastin and methotrexate, or any analog or derivative variant of the foregoing.

### 2. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### 3. Immunotherapy

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, *etc*.) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy, thus, could be used as part of a combined therapy, in conjunction with APF, APF receptor agonist, or APF receptor antagonist therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155.

### 4. Genes

In yet another embodiment, the secondary treatment is a secondary gene therapy in which a second therapeutic polynucleotide is administered before, after, or at the same time as an APF , APF receptor agonist, or APF receptor antagonist molecule, having a combined anti-hyperproliferative effect on target tissues. A variety of proteins are encompassed within the embodiment, including inhibitors of cellular proliferation, such as tumor suppressors, including p53; and/or regulators of programmed cell death, such as Bcl-2, for example.

### 5. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment disclosed herein, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the therapies disclosed herein may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### 6. Other agents

It is contemplated that other agents may be used in combination with agents identified using the present invention to improve the therapeutic efficacy of treatment. These additional agents include immunomodulatory agents, agents that affect the upregulation of cell surface receptors and GAP junctions, cytostatic and differentiation agents, inhibitors of cell adehesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abilities of agents identified using the present invention by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present invention to improve the anti-hyerproliferative efficacy of the treatments. Inhibitors of cell adehesion are contemplated to improve the efficacy. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination to improve the treatment efficacy.

Hormonal therapy may also be used in conjunction with agents identified using the present invention or in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

### XIV. Screening For Modulators Of APF and/or APF Receptor

The present invention comprises methods for identifying modulators of the function of APF or APF receptor as defined in the claims. Although these screening methods may be employed for modulators of APF or APF receptor, for the sake of brevity the following text will refer to modulators of APF receptor. These assays may comprise random screening of large libraries of candidate substances; alternatively, the assays may be used to focus on particular classes of compounds selected with an eye towards structural attributes that are believed to make them more likely to modulate the function of APF receptor. For example, perusal of the APF receptor, such as by x-ray crystallography or NMR studies, may identify one or more particular domains that the APF receptor of the assay employs.

By function, it is meant that one may assay for the activity of an APF receptor, such as binding of APF to APF receptor, for example.

To identify an APF receptor modulator, one generally will determine the function of APF receptor in the presence and absence of the candidate substance, a modulator defined as any substance that alters function. For example, a method generally comprises:

(a) providing a candidate modulator;

(b) admixing the candidate modulator with a compound or cell, or a suitable experimental animal, including an isolated compound or cell, or a suitable experimental animal;

(c) measuring one or more characteristics of the compound, cell or animal in step (b); and

(d) comparing the characteristic measured in step (c) with the characteristic of the compound, cell or animal in the absence of said candidate modulator,

wherein a difference between the measured characteristics indicates that said candidate modulator is, indeed, a modulator of the compound, cell or animal.

In certain aspects, there is a method of screening for a modulator of antiproliferative factor (APF) activity or APF receptor activity, comprising
(a) providing a candidate modulator;
(b) admixing the candidate modulator with APF, APF receptor-binding congener of APF, APF receptor, and/or APF receptor derivative;
(c) measuring one or more characteristics of APF, APF analog, APF receptor, or APF receptor analog; and
(d) comparing the characteristic measured in step (c) with the characteristic of the respective APF, APF receptor-binding congener of APF, APF receptor, or APF receptor analog in the absence of said candidate modulator,
   wherein a difference between the measured characteristics indicates that said candidate modulator:
   1) enhances binding of APF to its receptor; or
   2) inhibits binding of APF to its receptor

Assays may be conducted in cell free systems, in isolated cells, or in organisms including transgenic animals. In certain cases, assays are employed wherein candidate compounds that bind to APF receptor are assayed for ability to inhibit cell growth and/or for the ability to cause cell death.

It will, of course, be understood that all the screening methods of the present invention are useful in themselves notwithstanding the fact that effective candidates may not be found. The invention provides methods for screening for such candidates, not solely methods of finding them.

### A. Modulators

As used herein the term "candidate substance" refers to any molecule that may potentially inhibit or enhance APF activity, APF receptor activity, or APF interaction with its receptor. The candidate substance may be a sugar, a protein or fragment thereof, a small molecule, a nucleic acid molecule, or a mixture of combination thereof, for example. It may prove to be the case that the most useful pharmacological compounds will be compounds that are structurally related to APF or CKAP4. Using lead compounds to help develop improved compounds is know as "rational drug design" and includes not only comparisons with known inhibitors and activators, but predictions relating to the structure of target molecules.

The goal of rational drug design is to produce structural analogs of biologically active polypeptides or target compounds. By creating such analogs, it is possible to fashion drugs, which are more active or stable than the natural molecules, that have different susceptibility to alteration or that may affect the function of various other molecules. In one approach, one would generate a three-dimensional structure for a target molecule, or a fragment thereof. This could be accomplished by x-ray crystallography, computer modeling, by far-western, or by a combination thereof, for example.

It also is possible to use antibodies to ascertain the structure of a target compound activator or inhibitor. In principle, this approach yields a pharmacore upon which subsequent drug design can be based. It is possible to bypass protein crystallography altogether by generating anti-idiotypic antibodies to a functional, pharmacologically active antibody. As a mirror image of a mirror image, the binding site of anti-idiotype would be expected to be an analog of the original antigen. The anti-idiotype could then be used to identify and isolate peptides from banks of chemically- or biologically-produced peptides. Selected peptides would then serve as the pharmacore. Anti-idiotypes may be generated using the methods described herein for producing antibodies, using an antibody as the antigen.

On the other hand, one may simply acquire, from various commercial sources, small molecule libraries that are believed to meet the basic criteria for useful drugs in an effort to "brute force" the identification of useful compounds. Screening of such libraries, including combinatorially generated libraries (e.g., peptide libraries), is a rapid and efficient way to screen large number of related (and unrelated) compounds for activity. Combinatorial approaches also lend themselves to rapid evolution of potential drugs by the creation of second, third and fourth generation compounds modeled of active, but otherwise undesirable compounds.

Candidate compounds may include fragments or parts of naturally-occurring compounds, or may be found as active combinations of known compounds, which are otherwise inactive. It is proposed that compounds isolated from natural sources, such as animals, bacteria, fungi, plant sources, including leaves and bark, and marine samples may be assayed as candidates for the presence of potentially useful pharmaceutical agents. It will be understood that the pharmaceutical agents to be screened could also be derived or synthesized from chemical compositions or man-made compounds. Thus, it is understood that the candidate substance identified by the present invention may be peptide, polypeptide, polynucleotide, small molecule inhibitors or any other compounds that may be designed through rational drug design starting from known inhibitors or stimulators.

Other suitable modulators include antisense molecules, ribozymes, and antibodies (including single chain antibodies), each of which would be specific for the target molecule. Such compounds are described in greater detail elsewhere in this document. For example, an antisense molecule that bound to a translational or transcriptional start site, or splice junctions, would be ideal candidate inhibitors.

In addition to the modulating compounds initially identified, the inventors also contemplate that other sterically similar compounds may be formulated to mimic the key portions of the structure of the modulators. Such compounds, which may include peptidomimetics of peptide modulators, may be used in the same manner as the initial modulators.

An inhibitor may be one which exerts its inhibitory or activating effect upstream, downstream or directly on APF receptor. Regardless of the type of inhibitor or activator identified by the present screening methods, the effect of the inhibition or activator by such a compound results in modulation of APF receptor as compared to that observed in the absence of the added candidate substance.

### B. In vitro Assays

A quick, inexpensive and easy assay to run is an *in vitro* assay. Such assays generally use isolated molecules, can be run quickly and in large numbers, thereby increasing the amount of information obtainable in a short period of time. A variety of vessels may be used to run the assays, including test tubes, plates, dishes and other surfaces such as dipsticks or beads.

One example of a cell free assay is a binding assay. While not directly addressing function, the ability of a modulator to bind to a target molecule in a specific fashion is strong evidence of a related biological effect. For example, binding of a molecule to a target may, in and of itself, be inhibitory, due to steric, allosteric or charge-charge interactions. The target may be either free in solution, fixed to a support, expressed in or on the surface of a cell. Either the target or the compound may be labeled, thereby permitting determining of binding. Usually, the target will be the labeled species, decreasing the chance that the labeling will interfere with or enhance binding. Competitive binding formats can be performed in which one of the agents is labeled, and one may measure the amount of free label versus bound label to determine the effect on binding.

A technique for high throughput screening of compounds is described in WO 84/03564. Large numbers of small peptide test compounds are synthesized on a solid substrate, such as plastic pins or some other surface. Bound polypeptide is detected by various methods.

### C. In cyto Assays

The screening of compounds for their ability to modulate APF receptor in cells is also contemplated. Various cell lines can be utilized for such screening assays, including cells specifically engineered for this purpose. Exemplary cells include, for example, bladder cells, such as bladder epithelial cells, cancer cells, and so forth.

Depending on the assay, culture may be required. The cell is examined using any of a number of different physiologic assays. Alternatively, molecular analysis may be performed, for example, looking at protein expression, mRNA expression (including differential display of whole cell or polyA RNA) and others.

### D. In vivo Assays

*In vivo* assays involve the use of various animal models, including transgenic animals that have been engineered to have specific defects, or carry markers that can be used to measure the ability of a candidate substance to reach and effect different cells within the organism. Due to their size, ease of handling, and information on their physiology and genetic make-up, mice are a preferred embodiment, especially for transgenics. However, other animals are suitable as well, including rats, rabbits, hamsters, guinea pigs, gerbils, woodchucks, cats, dogs, sheep, goats, pigs, cows, horses and monkeys (including chimps, gibbons and baboons). Assays for modulators may be conducted using an animal model derived from any of these species.

In such assays, one or more candidate substances are administered to an animal, and the ability of the candidate substance(s) to alter one or more characteristics, as compared to a similar animal not treated with the candidate substance(s), identifies a modulator. The characteristics may be any of those discussed above with regard to the function of a particular compound (*e.g*., enzyme, receptor, hormone) or cell (*e.g.,* growth, tumorigenicity, survival), or instead a broader indication such as behavior, anemia, immune response, *etc.*

The present invention provides methods of screening for a candidate substance that modulates APF receptor as defined in the claims. In these embodiments, the present invention is directed to a method for determining the ability of a candidate substance to interfere with or enhance binding of APF to APF receptor, generally including the steps of: administering a candidate substance to the animal; and determining the ability of the candidate substance to reduce one or more characteristics of APF receptor, such as receptor binding, or enhance one or more characteristics of APF receptor, such as receptor binding.

Treatment of these animals with test compounds will involve the administration of the compound, in an appropriate form, to the animal. Administration will be by any route that could be utilized for clinical or non-clinical purposes, including but not limited to oral, nasal, buccal, or even topical. Alternatively, administration may be by intratracheal instillation, bronchial instillation, intradermal, subcutaneous, intramuscular, intraperitoneal or intravenous injection. Specifically contemplated routes are systemic intravenous injection, regional administration *via* blood or lymph supply, or directly to an affected site.

Determining the effectiveness of a compound *in vivo* may involve a variety of different criteria. Also, measuring toxicity and dose response can be performed in animals in a more meaningful fashion than in *in vitro* or *in cyto* assays.

### XV. Biological Functional Equivalents

### A. Modified Polypeptides

The biological functional equivalent may comprise a polynucleotide that has been engineered to comprise distinct sequences while at the same time retaining the capacity to encode the "wild-type" or standard APF receptor protein (or the peptide component of APF). This can be accomplished to the degeneracy of the genetic code, *i.e*., the presence of multiple codons, which encode for the same amino acids. In one example, one of skill in the art may wish to introduce a restriction enzyme recognition sequence into a polynucleotide while not disturbing the ability of that polynucleotide to encode a protein.

In another example, a polynucleotide may be (and encode) a biological functional equivalent with more significant changes. Certain amino acids may be substituted for other amino acids in a protein structure without appreciable loss of interactive binding capacity with structures such as, for example, antigen-binding regions of antibodies, binding sites on substrate molecules, receptors, and such like. So-called "conservative" changes do not disrupt the biological activity of the protein, as the structural change is not one that impinges of the protein's ability to carry out its designed function. It is thus contemplated by the inventors that various changes may be made in the sequence of polynucleotides, polypeptides, and peptides disclosed herein.

In terms of functional equivalents, it is well understood by the skilled artisan that, inherent in the definition of a "biologically functional equivalent" protein and/or polynucleotide, is the concept that there is a limit to the number of changes that may be made within a defined portion of the molecule while retaining a molecule with an acceptable level of equivalent biological activity. Biologically functional equivalents are thus defined herein as those proteins (and polynucleotides) in selected amino acids (or codons) may be substituted. Functional activity may be defined as the ability for APF to bind APF receptor.

In general, the shorter the length of the molecule, the fewer changes that can be made within the molecule while retaining function. Longer domains may have an intermediate number of changes. The full-length protein will have the most tolerance for a larger number of changes. However, it must be appreciated that certain molecules or domains that are highly dependent upon their structure may tolerate little or no modification.

In still other examples, a polypeptide is modified such that the activity of the molecule is altered. For example, an APF receptor may be modified such that it binds APF but does not retain any suitable function to act downstream thereof. An APF molecule may be modified such that it no longer binds the APF receptor, in certain aspects.

Amino acid substitutions are generally based on the relative similarity of the amino acid side-chain substituents, for example, their hydrophobicity, hydrophilicity, charge, size, and/or the like. An analysis of the size, shape and/or type of the amino acid side-chain substituents reveals that arginine, lysine and/or histidine are all positively charged residues; that alanine, glycine and/or serine are all a similar size; and/or that phenylalanine, tryptophan and/or tyrosine all have a generally similar shape. Therefore, based upon these considerations, arginine, lysine and/or histidine; alanine, glycine and/or serine; and/or phenylalanine, tryptophan and/or tyrosine; are defined herein as biologically functional equivalents.

To effect more quantitative changes, the hydropathic index of amino acids may be considered. Each amino acid has been assigned a hydropathic index on the basis of their hydrophobicity and/or charge characteristics, these are: isoleucine (+4.5); valine (+4.2); leucine (+3.8); phenylalanine (+2.8); cysteine/cystine (+2.5); methionine (+1.9); alanine (+1.8); glycine ( 0.4); threonine ( 0.7); serine ( 0.8); tryptophan ( 0.9); tyrosine ( 1.3); proline ( 1.6); histidine ( 3.2); glutamate ( 3.5); glutamine ( 3.5); aspartate ( 3.5); asparagine ( 3.5); lysine ( 3.9); and/or arginine ( 4.5).

The importance of the hydropathic amino acid index in conferring interactive biological function on a protein is generally understood in the art (Kyte & Doolittle, 1982, incorporated herein by reference). It is known that certain amino acids may be substituted for other amino acids having a similar hydropathic index and/or score and/or still retain a similar biological activity. In making changes based upon the hydropathic index, the substitution of amino acids whose hydropathic indices are within ±2 is preferred, those which are within ±1 are particularly preferred, and/or those within ±0.5 are even more particularly preferred.

It also is understood in the art that the substitution of like amino acids can be made effectively on the basis of hydrophilicity, particularly where the biological functional equivalent protein and/or peptide thereby created is intended for use in immunological embodiments. U.S. Patent 4,554,101,
states that the greatest local average hydrophilicity of a protein, as governed by the hydrophilicity of its adjacent amino acids, correlates with its immunogenicity and/or antigenicity, i.e., with a biological property of the protein.

As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine ( 0.4); proline (-0.5 ± 1); alanine ( 0.5); histidine ( 0.5); cysteine ( 1.0); methionine ( 1.3); valine ( 1.5); leucine ( 1.8); isoleucine ( 1.8); tyrosine ( 2.3); phenylalanine ( 2.5); tryptophan ( 3.4). In making changes based upon similar hydrophilicity values, the substitution of amino acids whose hydrophilicity values are within ±2 is preferred, those which are within ±1 are particularly preferred, and/or those within ±0.5 are even more particularly preferred.

### B. Altered Amino Acids

The present invention, in many aspects, relies on the synthesis of peptides and polypeptides *in cyto, via* transcription and translation of appropriate polynucleotides. These peptides and polypeptides will include the twenty "natural" amino acids, and post-translational modifications thereof. However, *in vitro* peptide synthesis permits the use of modified and/or unusual amino acids. A table of exemplary, but not limiting, modified and/or unusual amino acids is provided herein below.

| Table 1 - Modified and/or Unusual Amino Acids | | | |
|---|---|---|---|
| Abbr. | Amino Acid | Abbr | Amino Acid |
| Aad | 2-Aminoadipic acid | EtAsn | N-Ethylasparagine |
| BAad | 3- Aminoadipic acid | Hyl | Hydroxylysine |
| BAla | beta-alanine, beta-Amino-propionic acid | AHyl | allo-Hydroxylysine |
| Abu | 2-Aminobutyric acid | 3Hyp | 3-Hydroxyproline |
| 4Abu | 4- Aminobutyric acid, piperidinic acid | 4Hyp | 4-Hydroxyproline |
| Acp | 6-Aminocaproic acid | Ide | Isodesmosine |
| Ahe | 2-Aminoheptanoic acid | Aile | allo-Isoleucine |
| Aib | 2-Aminoisobutyric acid | MeGly | N-Methylglycine, sarcosine |
| BAib | 3-Aminoisobutyric acid | Melle | N-Methylisoleucine |
| Apm | 2-Aminopimelic acid | MeLys | 6-N-Methyllysine |
| Dbu | 2,4-Diaminobutyric acid | MeVal | N-Methylvaline |
| Des | Desmosine | Nva | Norvaline |
| Dpm | 2,2'-Diaminopimelic acid | Nle | Norleucine |
| Dpr | 2,3-Diaminopropionic acid | Om | Ornithine |
| EtGly | N-Ethylglycine | | |

### C. Mimetics

In addition to the biological functional equivalents discussed above, the present inventors also contemplate that structurally similar compounds may be formulated to mimic the key portions of peptide or polypeptides of the present invention. Such compounds, which may be termed peptidomimetics, may be used in the same manner as the peptides described herein and, hence, also are functional equivalents.

Certain mimetics that mimic elements of protein secondary and tertiary structure are described in Johnson *et al.* (1993). The underlying rationale behind the use of peptide mimetics is that the peptide backbone of proteins exists chiefly to orient amino acid side chains in such a way as to facilitate molecular interactions, such as those of antibody and/or antigen. A peptide mimetic is thus designed to permit molecular interactions similar to the natural molecule.

Some successful applications of the peptide mimetic concept have focused on mimetics of β-turns within proteins, which are known to be highly antigenic. Likely β turn structure within a polypeptide can be predicted by computer-based algorithms, as discussed herein. Once the component amino acids of the turn are determined, mimetics can be constructed to achieve a similar spatial orientation of the essential elements of the amino acid side chains.

Other approaches have focused on the use of small, multidisulfide-containing proteins as attractive structural templates for producing biologically active conformations that mimic the binding sites of large proteins. Vita *et al.* (1998). A structural motif that appears to be evolutionarily conserved in certain toxins is small (30-40 amino acids), stable, and high permissive for mutation. This motif is composed of a beta sheet and an alpha helix bridged in the interior core by three disulfides.

Beta II turns have been mimicked successfully using cyclic L-pentapeptides and those with D-amino acids. Weisshoff *et al.* (1999). Also, Johannesson *et al.* (1999) report on bicyclic tripeptides with reverse turn inducing properties.

Methods for generating specific structures have been disclosed in the art. For example, alpha-helix mimetics are disclosed in U.S. Patents 5,446,128; 5,710,245; 5,840,833; and 5,859,184. Theses structures render the peptide or protein more thermally stable, also increase resistance to proteolytic degradation. Six, seven, eleven, twelve, thirteen and fourteen membered ring structures are disclosed.

Methods for generating conformationally restricted beta turns and beta bulges are described, for example, in U.S. Patents 5,440,013; 5,618,914; and 5,670,155. Beta-turns permit changed side substituents without having changes in corresponding backbone conformation, and have appropriate termini for incorporation into peptides by standard synthesis procedures. Other types of mimetic turns include reverse and gamma turns. Reverse turn mimetics are disclosed in U.S. Patents 5,475,085 and 5,929,237, and gamma turn mimetics are described in U.S. Patents 5,672,681 and 5,674,976.

### XVI. Nucleic Acid-Based Expression Systems

In some embodiments, a nucleic acid-based expression system is employed, such as for encoding an agent that interferes with or enhances APF activity, APF receptor activity, or APF binding to APF receptor. The nucleic acid-based expression system may encode the agent and may be employed to deliver the agent to a cell. In certain embodiments, there is a nucleic acid, such as an expression construct, that encodes part or all of the APF receptor.

### A. Vectors

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g*., YACs). One of skill in the art would be well equipped to construct a vector through standard recombinant techniques (see, for example, Maniatis *et al.,* 1988 and Ausubel *et al.,* 1994.

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described infra.

### 1. Promoters and Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30 110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e*., containing different elements of different transcriptional regulatory regions, and/or mutations that alter expression. For example, promoters that are most commonly used in recombinant DNA construction include the β lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906 ). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB available on the world wide web) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

The identity of tissue-specific promoters or elements, as well as assays to characterize their activity, is well known to those of skill in the art.

### 2. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819 ).

### 3. Multiple Cloning Sites

Vectors can include a multiple cloning site (MCS), which is a nucleic acid region that contains multiple restriction enzyme sites, any of which can be used in conjunction with standard recombinant technology to digest the vector (see, for example, Carbonelli *et al.,* 1999, Levenson *et al.,* 1998, and Cocea, 1997.) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### 4. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, *Chandler et al.,* 1997).

### 5. Termination Signals

The vectors or constructs will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### 6. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal or the bovine growth hormone polyadenylation signal, convenient and known to function well in various target cells. Polyadenylation may increase the stability of the transcript or may facilitate cytoplasmic transport.

### 7. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### 8. Selectable and Screenable Markers

In certain embodiments cells containing a nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (tk) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### 9. Plasmid Vectors

In certain embodiments, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEMTM 11 may be utilized in making a recombinant phage vector which can be used to transform host cells, such as, for example, E. coli LE392.

Further useful plasmid vectors include pIN vectors (Inouye *et al.,* 1985); and pGEX vectors, for use in generating glutathione S transferase (GST) soluble fusion proteins for later purification and separation or cleavage. Other suitable fusion proteins are those with β-galactosidase, ubiquitin, and the like.

Bacterial host cells, for example, E. coli, comprising the expression vector, are grown in any of a number of suitable media, for example, LB. The expression of the recombinant protein in certain vectors may be induced, as would be understood by those of skill in the art, by contacting a host cell with an agent specific for certain promoters, *e.g.,* by adding IPTG to the media or by switching incubation to a higher temperature. After culturing the bacteria for a further period, generally of between 2 and 24 h, the cells are collected by centrifugation and washed to remove residual media.

### 10. Viral Vectors

The ability of certain viruses to infect cells or enter cells *via* receptor mediated endocytosis, and to integrate into host cell genome and express viral genes stably and efficiently have made them attractive candidates for the transfer of foreign nucleic acids into cells (*e.g*., mammalian cells). Compositions may be comprised in a viral vector that encode one or more agents that modulate APF receptor binding. Non-limiting examples of virus vectors that may be used to deliver a nucleic acid are described below.

### a. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell specific construct that has been cloned therein. Knowledge of the genetic organization or adenovirus, a 36 kb, linear, double stranded DNA virus, allows substitution of large pieces of adenoviral DNA with foreign sequences up to 7 kb (Grunhaus and Horwitz, 1992).

### b. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno associated virus (AAV) is an attractive vector system for use in the compositions of the present invention as it has a high frequency of integration and it can infect nondividing cells, thus making it useful for delivery of genes into mammalian cells, for example, in tissue culture (Muzyczka, 1992) or *in vivo*. AAV has a broad host range for infectivity (Tratschin *et al.,* 1984; Laughlin *et al.,* 1986; Lebkowski *et al.,* 1988; McLaughlin *et al.,* 1988). Details concerning the generation and use of rAAV vectors are described in U.S. Patent Nos. 5,139,941 and 4,797,368.

### c. Retroviral Vectors

Retroviruses have promise as delivery vectors due to their ability to integrate their genes into the host genome, transferring a large amount of foreign genetic material, infecting a broad spectrum of species and cell types and of being packaged in special cell lines (Miller, 1992).

In order to construct a retroviral vector, a nucleic acid (*e.g*., one encoding an agent of interest) is inserted into the viral genome in the place of certain viral sequences to produce a virus that is replication defective. In order to produce virions, a packaging cell line containing the *gag, pol,* and env genes but without the LTR and packaging components is constructed (Mann *et al.,* 1983). When a recombinant plasmid containing a cDNA, together with the retroviral LTR and packaging sequences is introduced into a special cell line (*e.g*., by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al*., 1975).

Lentiviruses are complex retroviruses, which, in addition to the common retroviral genes gag, pol, and env, contain other genes with regulatory or structural function. Lentiviral vectors are well known in the art (see, for example, Naldini *et al.,* 1996; Zufferey *et al*., 1997; Blomer *et al.,* 1997; U.S. Pat. Nos. 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiple attenuating the HIV virulence genes, for example, the genes env, vif, vpr, vpu and nef are deleted making the vector biologically safe.

Recombinant lentiviral vectors are capable of infecting non-dividing cells and can be used for both *in vivo* and ex *vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136 . One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

### d. Other Viral Vectors

Other viral vectors may be employed as expression constructs in the present invention. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### e. Delivery Using Modified Viruses

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes *via* sialoglycoprotein receptors.

Another approach to targeting of recombinant retroviruses was designed in which biotinylated antibodies against a retroviral envelope protein and against a specific cell receptor were used. The antibodies were coupled *via* the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

### B. Vector Delivery and Cell Transformation

Suitable methods for nucleic acid delivery for transformation of an organelle, a cell, a tissue or an organism for use with the current invention are believed to include virtually any method by which a nucleic acid (*e.g.,* DNA) can be introduced into an organelle, a cell, a tissue or an organism, as described herein or as would be known to one of ordinary skill in the art. Such methods include, but are not limited to, direct delivery of DNA such as by *ex vivo* transfection (Wilson *et al.,* 1989, Nabel et al, 1989), by injection (U.S. Patent Nos. 5,994,624, 5,981,274, 5,945,100, 5,780,448, 5,736,524, 5,702,932, 5,656,610, 5,589,466 and 5,580,859, microinjection (Harlan and Weintraub, 1985; U.S. Patent No. 5,789,215 ; by electroporation (U.S. Patent No. 5,384,253 ; Tur-Kaspa *et al.,* 1986; Potter *et al.,* 1984); by calcium phosphate precipitation (Graham and Van Der Eb, 1973; Chen and Okayama, 1987; Rippe *et al.,* 1990); by using DEAE dextran followed by polyethylene glycol (Gopal, 1985); by direct sonic loading (Fechheimer *et al.,* 1987); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by microprojectile bombardment (PCT Application Nos. WO 94/09699 and 95/06128; U.S. Patent Nos. 5,610,042; 5,322,783 5,563,055, 5,550,318, 5,538,877 and 5,538,880); by agitation with silicon carbide fibers (Kaeppler et al., 1990; U.S. Patent Nos. 5,302,523 and 5,464,765
); by Agrobacterium mediated transformation (U.S. Patent Nos. 5,591,616 and 5,563,055); by PEG mediated transformation of protoplasts (Omirulleh et al., 1993; U.S. Patent Nos. 4,684,611 and 4,952,500
); by desiccation/inhibition mediated DNA uptake (Potrykus *et al.,* 1985), and any combination of such methods. Through the application of techniques such as these, organelle(s), cell(s), tissue(s) or organism(s) may be stably or transiently transformed.

### 1. Ex vivo Transformation

Methods for tranfecting vascular cells and tissues removed from an organism in an *ex vivo* setting are known to those of skill in the art. For example, cannine endothelial cells have been genetically altered by retrovial gene tranfer *in vitro* and transplanted into a canine (Wilson *et al.,* 1989). In another example, yucatan minipig endothelial cells were tranfected by retrovirus *in vitro* and transplated into an artery using a double-ballonw catheter (Nabel *et al.,* 1989). Thus, it is contemplated that cells or tissues may be removed and tranfected *ex vivo* using nucleic acids In particular aspects, the transplanted cells or tissues may be placed into an organism. In preferred facets, a nucleic acid is expressed in the transplated cells or tissues.

### 2. Injection

In certain embodiments, a nucleic acid may be delivered to an organelle, a cell, a tissue or an organism *via* one or more injections (*i.e.,* a needle injection), such as, for example, subcutaneously, intradermally, intramuscularly, intervenously, intraperitoneally, *etc.* Methods of injection of vaccines are well known to those of ordinary skill in the art (*e.g.,* injection of a composition comprising a saline solution). Further embodiments of the present invention include the introduction of a nucleic acid by direct microinjection. Direct microinjection has been used to introduce nucleic acid constructs into Xenopus oocytes (Harland and Weintraub, 1985). The amount of agent used may vary upon the nature of the antigen as well as the organelle, cell, tissue or organism used

### 3. Electroporation

In certain embodiments, a nucleic acid is introduced into an organelle, a cell, a tissue or an organism *via* electroporation. Electroporation involves the exposure of a suspension of cells and DNA to a high voltage electric discharge. In some variants of this method, certain cell wall degrading enzymes, such as pectin degrading enzymes, are employed to render the target recipient cells more susceptible to transformation by electroporation than untreated cells (U.S. Patent No. 5,384,253
). Alternatively, recipient cells can be made more susceptible to transformation by mechanical wounding.

Transfection of eukaryotic cells using electroporation has been quite successful. Mouse pre B lymphocytes have been transfected with human kappa immunoglobulin genes (Potter *et al.,* 1984), and rat hepatocytes have been transfected with the chloramphenicol acetyltransferase gene (Tur Kaspa *et al.,* 1986) in this manner.

To effect transformation by electroporation in cells such as, for example, plant cells, one may employ either friable tissues, such as a suspension culture of cells or embryogenic callus or alternatively one may transform immature embryos or other organized tissue directly. In this technique, one would partially degrade the cell walls of the chosen cells by exposing them to pectin degrading enzymes (pectolyases) or mechanically wounding in a controlled manner. Examples of some species which have been transformed by electroporation of intact cells include maize (U.S. Patent No. 5,384,253; Rhodes *et al.,* 1995; D'Halluin *et al.,* 1992), wheat (Zhou *et al.,* 1993), tomato (Hou and Lin, 1996), soybean (Christou *et al.,* 1987) and tobacco (Lee *et al.,* 1989).

One also may employ protoplasts for electroporation transformation of plant cells (Bates, 1994; Lazzeri, 1995). For example, the generation of transgenic soybean plants by electroporation of cotyledon derived protoplasts is described by Dhir and Widholm in International Patent Application No. WO 9217598. Other examples of species for which protoplast transformation has been described include barley (Lazerri, 1995), sorghum (Battraw *et al.,* 1991), maize (Bhattacharjee *et al.,* 1997), wheat (He *et al*., 1994) and tomato (Tsukada, 1989).

### 4. Calcium Phosphate

In other embodiments, a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV 1, BHK, NIH3T3 and HeLa cells were transfected with a neomycin marker gene (Chen and Okayama, 1987), and rat hepatocytes were transfected with a variety of marker genes (Rippe *et al.,* 1990).

### 5. DEAE Dextran

In another embodiment, a nucleic acid is delivered into a cell using DEAE dextran followed by polyethylene glycol. In this manner, reporter plasmids were introduced into mouse myeloma and erythroleukemia cells (Gopal, 1985).

### 6. Sonication Loading

Additional embodiments include the introduction of a nucleic acid by direct sonic loading. LTK fibroblasts have been transfected with the thymidine kinase gene by sonication loading (Fechheimer *et al.,* 1987).

### 7. Liposome Mediated Transfection

In a further embodiment a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self rearrangement before the formation of closed structures and entrap water and dissolved solutes between the lipid bilayers (Ghosh and Bachhawat, 1991). Also contemplated is an nucleic acid complexed with Lipofectamine (Gibco BRL) or Superfect (Qiagen).

Liposome mediated nucleic acid delivery and expression of foreign DNA *in vitro* has been very successful (Nicolau and Sene, 1982; Fraley *et al.,* 1979; Nicolau *et al.,* 1987). The feasibility of liposome mediated delivery and expression of foreign DNA in cultured chick embryo, HeLa and hepatoma cells has also been demonstrated (Wong *et al.,* 1980).

In certain embodiments, a liposome may be complexed with a hemagglutinating virus (HVJ). This has been shown to facilitate fusion with the cell membrane and promote cell entry of liposome encapsulated DNA (Kaneda *et al.,* 1989). In other embodiments, a liposome may be complexed or employed in conjunction with nuclear non histone chromosomal proteins (HMG 1) (Kato *et al.,* 1991). In yet further embodiments, a liposome may be complexed or employed in conjunction with both HVJ and HMG 1. In other embodiments, a delivery vehicle may comprise a ligand and a liposome.

### C. Receptor Mediated Transfection

Still further, a nucleic acid may be delivered to a target cell *via* receptor mediated delivery vehicles. These take advantage of the selective uptake of macromolecules by receptor mediated endocytosis that will be occurring in a target cell. In view of the cell type specific distribution of various receptors, this delivery method adds another degree of specificity .

Certain receptor mediated gene targeting vehicles comprise a cell receptor specific ligand and a nucleic acid binding agent. Others comprise a cell receptor specific ligand to which the nucleic acid to be delivered has been operatively attached. Several ligands have been used for receptor mediated gene transfer (Wu and Wu, 1987; Wagner *et al.,* 1990; Perales *et al.,* 1994; Myers, EPO 0273085), which establishes the operability of the technique. Specific delivery in the context of another mammalian cell type has been described (Wu and Wu, 1993
). In certain aspects, a ligand will be chosen to correspond to a receptor specifically expressed on the target cell population.

In other embodiments, a nucleic acid delivery vehicle component of a cell specific nucleic acid targeting vehicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor mediated delivery of a nucleic acid to cells that exhibit upregulation of the EGF receptor.

In still further embodiments, the nucleic acid delivery vehicle component of a targeted delivery vehicle may be a liposome itself, which will preferably comprise one or more lipids or glycoproteins that direct cell specific binding. For example, lactosyl ceramide, a galactose terminal asialganglioside, have been incorporated into liposomes and observed an increase in the uptake of the insulin gene by hepatocytes (Nicolau *et al.,* 1987). It is contemplated that the tissue specific transforming constructs can be specifically delivered into a target cell in a similar manner.

### D. Microprojectile Bombardment

Microprojectile bombardment techniques can be used to introduce a nucleic acid into at least one, organelle, cell, tissue or organism (U.S. Patent No. 5,550,318; U.S. Patent No. 5,538,880; U.S. Patent No. 5,610,042; and PCT Application WO 94/09699 ). This method depends on the ability to accelerate DNA coated microprojectiles to a high velocity allowing them to pierce cell membranes and enter cells without killing them (Klein *et al.,* 1987). There are a wide variety of microprojectile bombardment techniques known in the art, many of which are applicable to the invention.

Microprojectile bombardment may be used to transform various cell(s), tissue(s) or organism(s), such as for example any plant species. Examples of species which have been transformed by microprojectile bombardment include monocot species such as maize (PCT Application WO 95/06128), barley (Ritala *et al.,* 1994; Hensgens *et al.,* 1993), wheat (U.S. Patent No. 5,563,055), rice (Hensgens *et al*., 1993), oat (Torbet *et al.,* 1995; Torbet *et al.,* 1998), rye (Hensgens *et al.,* 1993), sugarcane (Bower *et al.,* 1992), and sorghum (Casas *et al.,* 1993; Hagio *et al.,* 1991); as well as a number of dicots including tobacco (Tomes *et al.,* 1990; Buising and Benbow, 1994), soybean (U.S. Patent No. 5,322,783 ), sunflower (Knittel *et al*, 1994), peanut (Singsit *et al*., 1997), cotton (McCabe and Martinell, 1993), tomato (VanEck *et al.* 1995), and legumes in general (U.S. Patent No. 5,563,055).

In this microprojectile bombardment, one or more particles may be coated with at least one nucleic acid and delivered into cells by a propelling force. Several devices for accelerating small particles have been developed. One such device relies on a high voltage discharge to generate an electrical current, which in turn provides the motive force (Yang *et al.,* 1990). The microprojectiles used have consisted of biologically inert substances such as tungsten or gold particles or beads. Exemplary particles include those comprised of tungsten, platinum, and preferably, gold. It is contemplated that in some instances DNA precipitation onto metal particles would not be necessary for DNA delivery to a recipient cell using microprojectile bombardment. However, it is contemplated that particles may contain DNA rather than be coated with DNA. DNA coated particles may increase the level of DNA delivery *via* particle bombardment but are not, in and of themselves, necessary.

For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

An illustrative embodiment of a method for delivering DNA into a cell (*e.g*., a plant cell) by acceleration is the Biolistics Particle Delivery System, which can be used to propel particles coated with DNA or cells through a screen, such as a stainless steel or Nytex screen, onto a filter surface covered with cells, such as for example, a monocot plant cells cultured in suspension. The screen disperses the particles so that they are not delivered to the recipient cells in large aggregates. It is believed that a screen intervening between the projectile apparatus and the cells to be bombarded reduces the size of projectiles aggregate and may contribute to a higher frequency of transformation by reducing the damage inflicted on the recipient cells by projectiles that are too large.

### E. Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny. As used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells which do not contain a recombinantly introduced nucleic acid.

In certain embodiments, it is contemplated that RNAs or proteinaceous sequences may be co expressed with other selected RNAs or proteinaceous sequences in the same host cell. Co expression may be achieved by co transfecting the host cell with two or more distinct recombinant vectors. Alternatively, a single recombinant vector may be constructed to include multiple distinct coding regions for RNAs, which could then be expressed in host cells transfected with the single vector.

A tissue may comprise a host cell or cells to be transformed with an agent. The tissue may be part or separated from an organism. In certain embodiments, a tissue may comprise, but is not limited to, adipocytes, alveolar, ameloblasts, axon, basal cells, blood (*e.g*., lymphocytes), blood vessel, bone, bone marrow, brain, breast, cartilage, cervix, colon, cornea, embryonic, endometrium, endothelial, epithelial, esophagus, facia, fibroblast, follicular, ganglion cells, glial cells, goblet cells, kidney, liver, lung, lymph node, muscle, neuron, ovaries, pancreas, peripheral blood, prostate, skin, skin, small intestine, spleen, stem cells, stomach, testes, anthers, ascite tissue, cobs, ears, flowers, husks, kernels, leaves, meristematic cells, pollen, root tips, roots, silk, stalks, and all cancers thereof.

In certain embodiments, the host cell or tissue may be comprised in at least one organism. In certain embodiments, the organism may be, but is not limited to, a prokayote (*e.g*., a eubacteria, an archaea) or an eukaryote, as would be understood by one of ordinary skill in the art (see, for example, webpage http://phylogeny.arizona.edu/tree/phylogeny.html).

Numerous cell lines and cultures are available for use as a host cell, and they can be obtained through the American Type Culture Collection (ATCC), which is an organization that serves as an archive for living cultures and genetic materials. An appropriate host can be determined by one of skill in the art based on the vector backbone and the desired result. A plasmid or cosmid, for example, can be introduced into a prokaryote host cell for replication of many vectors. Cell types available for vector replication and/or expressioninclude, but are not limited to, bacteria, such as E. coli (*e.g*., E. coli strain RR1, E. coli LE392, E. coli B, E. coli X 1776 (ATCC No. 31537) as well as E. coli W3110 (F , lambda , prototrophic, ATCC No. 273325), DH5α, JM109, and KC8, bacilli such as Bacillus subtilis; and other enterobacteriaceae such as Salmonella typhimurium, Serratia marcescens, various Pseudomonas specie, as well as a number of commercially available bacterial hosts such as SURE® Competent Cells and SOLOPACK™ Gold Cells (STRATAGENE®, La Jolla). In certain embodiments, bacterial cells such as E. coli LE392 are particularly contemplated as host cells for phage viruses.

Examples of eukaryotic host cells for replication and/or expression of a vector include, but are not limited to, HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### F. Expression Systems

Numerous expression systems exist that comprise at least a part or all of the compositions discussed above. Prokaryote- and/or eukaryote-based systems can be employed for use with the present invention to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Patent No. 5,871,986, 4,879,236, and which can be bought, for example, under the name MAXBAC® 2.0 from INVITROGEN® and BACPACK™ BACULOVIRUS EXPRESSION SYSTEM FROM CLONTECH®.

Other examples of expression systems include STRATAGENE®'s COMPLETE CONTROL™ Inducible Mammalian Expression System, which involves a synthetic ecdysone-inducible receptor, or its pET Expression System, an E. coli expression system. Another example of an inducible expression system is available from INVITROGEN®, which carries the T-REX™ (tetracycline-regulated expression) System, an inducible mammalian expression system that uses the full-length CMV promoter. INVITROGEN® also provides a yeast expression system called the Pichia methanolica Expression System, which is designed for high-level production of recombinant proteins in the methylotrophic yeast Pichia methanolica. One of skill in the art would know how to express a vector, such as an expression construct, to produce a nucleic acid sequence or its cognate polypeptide, protein, or peptide.

It is contemplated that the proteins, polypeptides or peptides produced by the methods may be "overexpressed", *i.e.,* expressed in increased levels relative to its natural expression in cells. Such overexpression may be assessed by a variety of methods, including radio labeling and/or protein purification. However, simple and direct methods are preferred, for example, those involving SDS/PAGE and protein staining or western blotting, followed by quantitative analyses, such as densitometric scanning of the resultant gel or blot. A specific increase in the level of the recombinant protein, polypeptide or peptide in comparison to the level in natural cells is indicative of overexpression, as is a relative abundance of the specific protein, polypeptides or peptides in relation to the other proteins produced by the host cell and, *e.g*., visible on a gel.

In some embodiments, the expressed proteinaceous sequence forms an inclusion body in the host cell, the host cells are lysed, for example, by disruption in a cell homogenizer, washed and/or centrifuged to separate the dense inclusion bodies and cell membranes from the soluble cell components. This centrifugation can be performed under conditions whereby the dense inclusion bodies are selectively enriched by incorporation of sugars, such as sucrose, into the buffer and centrifugation at a selective speed. Inclusion bodies may be solubilized in solutions containing high concentrations of urea (*e.g*. 8M) or chaotropic agents such as guanidine hydrochloride in the presence of reducing agents, such as β mercaptoethanol or DTT (dithiothreitol), and refolded into a more desirable conformation, as would be known to one of ordinary skill in the art.

### XVII. Proteins, Polypeptides, and Peptides

The term "purified proteins, polypeptides, or peptides" as used herein, is intended to refer to an proteinaceous composition, isolatable from mammalian cells or recombinant host cells, wherein the at least one protein, polypeptide, or peptide is purified to any degree relative to its naturally obtainable state, *i.e.,* relative to its purity within a cellular extract. A purified protein, polypeptide, or peptide therefore also refers to a wild type or mutant protein, polypeptide, or peptide free from the environment in which it naturally occurs.

The nucleotide and protein, polypeptide and peptide sequences for various genes have been previously disclosed, and may be found at computerized databases known to those of ordinary skill in the art. One such database is the National Center for Biotechnology Information's GenBank® and GenPept® databases. The coding regions for these known genes may be amplified and/or expressed using the techniques disclosed herein or by any technique that would be know to those of ordinary skill in the art. Additionally, peptide sequences may be sythesized by methods known to those of ordinary skill in the art, such as peptide synthesis using automated peptide synthesis machines, such as those available from Applied Biosystems (Foster City, CA).

Generally, "purified" will refer to a specific protein, polypeptide, or peptide composition that has been subjected to fractionation to remove various other proteins, polypeptides, or peptides, and which composition substantially retains its activity, as may be assessed, for example, by the protein assays, as described herein below, or as would be known to one of ordinary skill in the art for the desired protein, polypeptide or peptide.

Where the term "substantially purified" is used, this will refer to a composition in which the specific protein, polypeptide, or peptide forms the major component of the composition, such as constituting about 50% of the proteins in the composition or more. In preferred embodiments, a substantially purified protein will constitute more than 60%, 70%, 80%, 90%, 95%, 99% or even more of the proteins in the composition.

A peptide, polypeptide or protein that is "purified to homogeneity," as applied to the present invention, means that the peptide, polypeptide or protein has a level of purity where the peptide, polypeptide or protein is substantially free from other proteins and biological components. For example, a purified peptide, polypeptide or protein will often be sufficiently free of other protein components so that degradative sequencing may be performed successfully.

Various methods for quantifying the degree of purification of proteins, polypeptides, or peptides will be known to those of skill in the art in light of the present disclosure. These include, for example, determining the specific protein activity of a fraction, or assessing the number of polypeptides within a fraction by gel electrophoresis.

To purify a desired protein, polypeptide, or peptide a natural or recombinant composition comprising at least some specific proteins, polypeptides, or peptides will be subjected to fractionation to remove various other components from the composition. In addition to those techniques described in detail herein below, various other techniques suitable for use in protein purification will be well known to those of skill in the art. These include, for example, precipitation with ammonium sulfate, PEG, antibodies and the like or by heat denaturation, followed by centrifugation; chromatography steps such as ion exchange, gel filtration, reverse phase, hydroxylapatite, lectin affinity and other affinity chromatography steps; isoelectric focusing; gel electrophoresis; and combinations of such and other techniques.

Another example is the purification of a specific fusion protein using a specific binding partner. Such purification methods are routine in the art. Any fusion protein purification method can now be practiced. This is exemplified by the generation of an specific protein glutathione **S** transferase fusion protein, expression in E. coli, and isolation to homogeneity using affinity chromatography on glutathione agarose or the generation of a polyhistidine tag on the bN or **C** terminus of the protein, and subsequent purification using Ni affinity chromatography. However, given many DNA and proteins are known, or may be identified and amplified using the methods described herein, any purification method can now be employed.

Although preferred for use in certain embodiments, there is no general requirement that the protein, polypeptide, or peptide always be provided in their most purified state. Indeed, it is contemplated that less substantially purified protein, polypeptide or peptide, which are nonetheless enriched in the desired protein compositions, relative to the natural state, will have utility in certain embodiments.

Methods exhibiting a lower degree of relative purification may have advantages in total recovery of protein product, or in maintaining the activity of an expressed protein. Inactive products also have utility in certain embodiments, such as, *e.g.,* in determining antigenicity *via* antibody generation.

### XVIII. Examples

The following Examples are included to demonstrate preferred embodiments of the invention. It should be appreciated by those of skill in the art that the techniques disclosed in the samples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### EXAMPLE 1

### AFFINITY PURIFICATION AND IDENTIFICATION OF APF-BINDING BLADDER EPITHELIAL CELL PROTEINS

Solubilized bladder epithelial cell membrane proteins that bound with high affinity to biotinylated-APF were eluted with 0.1% formic acid/20% acetonitrile. The eluate was resolved by denaturing sodium dodecylsulfate polyacrylamide gel electrophoresis (SDS-PAGE), and proteins were visualized by silver staining. Two protein bands were evident with molecular masses of approximately 63 and 54 kDa (FIG. 1A). These bands were excised, in-gel digested with trypsin, and the resulting tryptic fragments were analyzed by nanoflow reversed-phase liquid chromatography (nanoRPLC) coupled with a linear ion-trap mass spectrometer operating in a data-dependent tandem mass spectrometry (MS/MS) mode. This analysis resulted in the unequivocal identification of the 63 kDa band as cytoskeleton-associated protein 4/p63 (CKAP4/p63) (16 unique peptides,30% sequence coverage) and the 54 kDa band as vimentin (13 peptides, 28% sequence coverage) (FIG. 1B).

The identities of the 63 kDa and 54 kDa proteins were confirmed by Western blot using monoclonal antibodies specific for CKAP4/p63 and vimentin. As shown in FIG. 2, these antibodies bound specifically to the 63 kDa and 54 kDa proteins, respectively, that had been isolated by high affinity binding to biotinylated APF.

### EXAMPLE 2

### EVIDENCE FOR CKAP4 PROTEIN EXPRESSION ON CELL MEMBRANES OF NORMAL AND IC BLADDER EPITHELIAL CELL EXPLANTS

To establish that CKAP4 may indeed be a cell membrane receptor for APF required evidence that this protein is present on the cell membrane of bladder epithelial cells from both normal controls and IC patients. Cell membrane preparations from 4 IC patients and their age-, race- and gender-matched controls all expressed CKAP4/p63 protein on the cell membrane as determined by Western blot analysis (FIG. 3). Although the amount of CKAP4/p63 expression relative to beta actin expression varied between individuals, there was not a notable difference in expression between cells explanted from IC patients vs. controls.

### EXAMPLE 3

### INHIBITION OF APF ACTIVITY BY ANTI-CKAP4/P63 MONOCLONAL ANTIBODY

APF was discovered as a cell growth inhibitor from the urine of patients with IC. To establish whether CKAP4/p63 is a true receptor for APF, we determined whether antibodies that bind to CKAP4/p63 prevent the antiproliferative effects of APF on bladder epithelial cells. Normal human bladder epithelial cells were preincubated with monoclonal anti-CKAP4/p63 antibodies for 1.5 hours at 37 C prior to APF exposure. As shown in FIG. 4, preincubation with anti-CKAP4/p63 antibodies decreased the sensitivity of normal bladder epithelial cells to APF in a dose-dependent manner. In comparison, preincubation with the same concentrations of an isotype control antibody directed against an unrelated cell protein did not inhibit APF activity.

### EXAMPLE 4

### siRNA KNOCKDOWN OF CKAP4/P63

Additional evidence that CKAP4/p63 is a functional cell membrane receptor for APF was provided by transient transfection of cells with siRNA for CKAP4/p63 prior to APF exposure. Electroporation of normal bladder epithelial cells with double-stranded siRNA for CKAP4/p63 resulted in decreased protein expression of CKAP4/p63 relative to beta actin (FIG. 5A) as well as approximately one thousand-fold decreased sensitivity of the cells to APF (FIG. 5B). In comparison, control cells electroporated without siRNA or with a scrambled siRNA had little or no change in CKAP4/p63 protein expression relative to beta actin, and no decrease in sensitivity to APF compared to negative control cells that did not undergo electroporation.

### EXAMPLE 5

### CONFOCAL IMMUNOFLUORESCENCE MICROSCOPY OF CKAP4 AND RHODAMINE-LABELED APF BINDING IN BLADDER EPITHELIAL CELLS

To determine whether APF binding co-localizes with CKAP4/p63 in cells, normal bladder epithelial cells were fixed and incubated with anti-CKAP4 monoclonal antibodies and a FTTC-labeled secondary antibody, followed by rhodamine-labeled APF. The rhodamine-labeled APF congener was determined to have similar (approximately 85%) antiproliferative activity to synthetic, unlabeled APF on normal bladder epithelial cells. As shown in FIG. 6, the distribution of anti-CKAP4/p63 binding (FIG. 6A) overlapped with rhodamine-labeled APF binding (FIG. 6B), with both entities present on the cell membrane as well as concentrated in the perinuclear cytoplasm (FIG. 6C). Controls with secondary FITC-labeled anti-mouse antibody alone were negative for fluorescent signal.

### EXAMPLE 6

### SIGNIFICANCE OF THE PRESENT INVENTION

Demonstrated is that CKAP4/p63 is the functional bladder epithelial cell receptor for APF, an inhibitor of cell proliferation secreted from bladder epithelial cells in patients suffering from the chronic painful bladder disorder called interstitial cystitis. CKAP4/p63 was isolated by affinity binding to APF, and its identity was determined by mass spectrometry and confirmed by Western blot analysis. The role of CKAP4/p63 in mediating APF's activity was determined by showing that both anti-CKAP4 antibodies and siRNA knockdown of CKAP4/p63 expression block the effects of APF on normal bladder epithelial cell proliferation.

APF causes specific changes in normal bladder epithelial cells that mimic changes seen in cells from IC patients *in vitro,* including profoundly inhibited cell proliferation (Keay *et al.,* 2000; Keay *et al.,* 1996) decreased production of heparin-binding epidermal growth factor-like growth factor (HB-EGF) *(Keay et al.,* 2000), a specifically altered gene expression pattern 8, and decreased tight junction formation with increased bladder epithelial cell monolayer permeability (Zhang *et al.,* 2005). Because decreased urine levels of HB-EGF (Keay *et al.,* 1997), bladder epithelial thinning or ulceration 3,4, abnormal expression of some of the same proteins (Slobodov *et al.,* 2004), and bladder epithelial leakiness (Parsons *et al.,* 1991), have all been described in IC patients *in vivo*, APF appears to play a pivotal role in the pathogenesis of IC. Identification of a receptor for APF on human bladder epithelial cells will therefore advance understanding of the mechanism of pathogenesis for IC, as well as aid in the development of specific therapies for this disorder.

The unique nature of APF as the first small sialoglycopeptide growth inhibitor to be completely characterized, and the first secreted frizzled-related protein to be identified that contains a transmembrane region of a frizzled protein exclusively, suggest the intriguing possibility that other such negative growth regulators may exist, either in association with normal embryonic development or with other disease states in which tissue development is impaired. Two other natural sialoglycopeptide growth inhibitors have been identified but have yet to be completely characterized (Auger *et al.,* 1989; Fattaey *et al.,* 1997), one of which is known to cause reversible growth inhibition like APF (Fattaey *et al.,* 1997; Keay *et al.,* 2003). However, the relationship of either of these negative growth factors to APF or frizzled proteins is unknown at this time.

The discovery of APF and its association with the CKAP4/p63 receptor provide several lessons in biomarker discovery. APF activity was first noted to be present in urine from IC patients (Keay *et al.,* 1996); the subsequent determination that this factor was probably made or activated in the distal urinary tract of these patients 16 lead to the discovery that APF appears to be made exclusively by explanted bladder cells from IC patients but not from controls (Keay *et al.,* 2000). Much of the current focus in the discovery of novel biomarkers leverages technologies that acquire large amounts of data (*e.g*. mass spectrometry and mRNA arrays) and topically search for changes in the abundance of wild-type proteins between disease-affected persons compared to normal controls (Zhou *et al*., 2005). Although mass spectrometry was instrumental in determining the structure of purified APF (Keay *et al*., 2004), in specific embodiments the approach of using biological activity to identify substances that are pathogenetically related to disease may be more fruitful, particularly for abnormal, disease-specific proteins. The identification of such proteins using proteomics alone can be difficult because disease-specific biomarkers are often present at much lower (nanomolar to picomolar) concentrations than other proteins (Alaiya *et al.,* 2005), and the structure and presence of APF in any cell or body fluid could not have been predicted or identified based on presently annotated genomic and proteomic databases. However, based on the high activity of APF and its specificity for urine samples and cells from IC patients, it may represent a novel class of small, modified bioactive peptides that can also function as disease-specific biomarkers.

CKAP4/p63 was first described as a reversibly palmitoylated type II transmembrane receptor (also called CLIMP-63) and was originally localized to the rough endoplasmic reticulum (ER) of fibroblast-like cells, epithelial cells, and plasma cells (Schweizer *et al.,* 1995; Banham *et al.,* 1997). It has recently been shown to be a functional vascular smooth muscle cell membrane receptor for tissue plasminogen activator (tPA) (Razzaq *et al.,* 2003) and is one of several ER transmembrane proteins known to also localize at the cell membrane (Tran *et al.,* 2002; Okazaki *et al.,* 2000). CKAP4/p63 has been shown to be present also in several types of epithelial cells (including COS and HeLa cells) where it helps to anchor the rough ER to microtubules (Klopfenstein *et al.,* 1998), a function that requires direct interaction of the cytoplasmic tail of the receptor to microtubules (Klopfenstein *et al.,* 1998) and is regulated by CKAP4/p63 phosphorylation (Vedrenne *et al.,* 2005).

Although the association between CKAP4/p63 and the cytoskeleton has been demonstrated (Vedrenne *et al.,* 2005), a specific association with vimentin has not previously been shown. The significance of CKAP4/p63 isolating with vimentin, which is expressed in high concentrations in basilar but not terminally differentiated bladder epithelial cells (Danahey *et al.,* 1995), is unknown. It is interesting to note, however, that vimentin was one of only nine bladder epithelial cell proteins shown to be downregulated in IC (vs. normal) or APF-treated (vs. Mock APF-treated) bladder epithelial cells in our previous microarray studies of the mRNA expression of approximately 4000 genes and Western blot analysis of specific cytoskeletal and tight junction protein expression *(Keay et al.,* 2003; Zhang *et al.,* 2005). Although the Western blot data in these Examples indicate no significant difference in the amount of CKAP4/p63 total protein made by IC and normal bladder cells, whether APF binding affects the binding of CKAP4/p63 to the cytoskeleton, or its distribution in bladder epithelial cells, may be determined by methods known in the art.

### EXAMPLE 7

### EXEMPLARY METHODS AND REAGENTS

The present invention concerns exemplary methods and reagents that may be employed in the invention, although one of skill in the art is aware of other suitable methods and reagents that may be utilized as an alternative or in addition.

### Cell Cultures

Explanted bladder epithelial cells were propagated from biopsies of 4 patients who had undergone cystoscopy and fulfilled the NIDDK/NIH diagnostic criteria for interstitial cystitis (Gillenwater and Wein, 1987) and their age-, race- and gender-matched controls. These cells were grown from the biopsies using DMEM-F12 (Mediatech, Herndon, VA) with 10% heat-inactivated FBS, 1 % antibiotic/antimycotic solution, 1 % L-glutamine, 0.25 units/ml insulin (all from Sigma, St. Louis, MO), and 5 ng/ml recombinant human epidermal growth factor (EGF) (R & D Systems, Minneapolis, MN) at 37 °C in a 5% CO2 atmosphere, and characterized by binding of AE-1/AE-3 pancytokeratin antibodies (Signet, Dedham, MA), as previously described 1(Keay *et al.,* 2004; Keay *et al.,* 2000). All patients were at least 18 years old and enrolled in accordance with guidelines of the Institutional Review Board of the University of Maryland School of Medicine.

### Biotinylated Synthetic APF

APF biotinylated on the C-terminal lysine residue was synthesized up to the N-terminal valine by solid phase methods on a Nautilus 2400 synthesizer (Argonaut Technologies, Foster City, CA) utilizing standard Fmoc chemistry on N-α-Fmoc-N-ε-biotinyl-L-lysinyl 2-chlorotrityl resin. Fmoc-protected L amino acids (Novabiochem) were coupled using HATU (Sigma-Aldrich) and HOAt (Anaspec) reagents. The Fmoc-protected Galβ1-3GalNAcα-O-threonine was then coupled to the remaining peptide backbone, as previously described (Keay *et al.,* 2004). After purification by HPLC on a C8 column, the identity of the biotinylated synthetic APF was confirmed by mass spectrometry, and its antiproliferative activity confirmed in primary normal bladder epithelial cells (this congener has approximately 80% activity as compared to the parent synthetic GalNAcβ1-3Gal-APF congener).

### Receptor Purification

Explanted bladder epithelial cells were grown in DMEM-F12 with supplements as described above until confluent, and then cultured in serum-free MEM medium (containing only 1% L-glutamine and 1% antibiotic/antimycotic solution) for 48 hours at 37 °C, rinsed with cold phosphate-buffered saline (PBS) in the culture flasks, scraped with sterile cell scrapers into ice cold PBS, pelleted by low speed centrifugation at 4 °C, and then immediately frozen at -80 °C. The frozen pellet (containing 3 x 10⁷ cells) was then washed three times with 0.5 ml ice cold wash buffer [phosphate-buffered saline (PBS), pH 7.2, 1 mM NaVO₃, 10 mM NaF, 1 mM EDTA, 0.1 mM phenylmethylsulfonyl fluoride (PMSF)] and repelleted by centrifugation for 15 min at 5000 x g. Cells were then further resuspended in 0.5 ml ice cold wash buffer and sonicated with a microtip sonifier (Branson Digital Sonifier, model 250, Branson Ultrasonics, Corp, Danbury, CT) for 30 seconds at 20% power on ice. Sonication was performed three times allowing for a 5 minute cool down on ice between sonic bursts. The cell lysate was centrifuged at 15000 x g for 30 minutes at 4 °C to pellet the microsomal fraction. The pellet was then washed three times by resuspending in 0.5 ml wash buffer followed by centrifugation at 15000 x g for 30 minutes. The microsomal fraction was finally solubilized in PBS, pH 7.2 containing 1 mM NaVO₃, 10 mM NaF, 1 mM EDTA, 0.1 mM PMSF and 1% Triton X-100. This solution was allowed to incubate on ice for 30 minutes and diluted to 0.5% Triton X-100 with ice cold wash buffer.

The solubilized protein fraction was then loaded onto an APF-bound affinity spin column generated by immobilizing synthetic biotinylated APF to a streptavidin-agarose bead stationary phase support (400 ml bed volume, Pierce Biotechnology, Rockford, IL). The column was washed 6 times with 500 ml of PBS, pH 7.2 containing 1 mM NaVO₃. 10 mM NaF, 1 mM EDTA, 0.1 mM PMSF and 0.2% Triton X-100 where each wash contained an increasing concentration of NaCl as follows: 10 mM, 100 mM, 250mM, 500 mM, and 1 M NaCl. The remaining bound proteins were eluted with 1% formic acid containing 0.2% Triton X-100. Each of the eluates were lyophilized to dryness, resuspended in Laemmli buffer, boiled for 5 minutes and resolved by one dimensional (ID) sodium dodecyl sulfate (SDS) polyacrylamide gel electrophoresis (PAGE) followed by visualization by silver stain. Sections were cut from the 1D SDS-PAGE gel containing the protein bands uniquely isolated by high affinity binding to the immobilized APF (as compared to the control isolations), and proteins in these slices were digested according to Wilm *et al.* (1996). The extracted tryptic peptides were then analyzed by nanoflow reversed-phase liquid chromatography (nanoRPLC) coupled online with tandem mass spectrometry (MS/MS).

### Nanoflow Reversed-Phase Liquid Chromatography-Tandem Mass Spectrometry

Chromatographic separations of the tryptic peptides were conducted using a microcapillary column with an integrated electrospray ionization (ESI) emitter constructed by flame pulling a fine tip (~ 5-7 mm orifice) on a 75 mm inner diameter x 360 mm outer diameter x 10 cm long segment of fused silica (Polymicro Technologies Inc., Phoenix, AZ). This integrated ESI column was slurry packed in-house with 5 mm, 300 Å pore size C-18 stationary phase (Jupiter, Phenomenex, Torrance, CA). The integrated ESI column was connected *via* a stainless steel zero dead volume union to an Agilent 1100 nanoflow LC system (Agilent Technologies, Palo Alto, CA), coupled online to a linear ion-trap (LTT) mass spectrometer (LTQ, ThermoElectron, Inc., San Jose, CA). After sample injection, a 20 minute wash with 98% mobile phase A (0.1% HCOOH in water) was applied and peptides were eluted using a linear gradient of 2% mobile phase B (0.1% HCOOH in CH₃CN) to 42% mobile phase B over 40 minutes with a constant flow rate of 200 nl/min. The column was washed for 15 min with 98% mobile phase B and re-equilibrated with 98% mobile phase A prior to subsequent sample loading.

The integrated nanoRPLC ESI column was coupled online to an LIT-MS using the manufacturer's nanoelectrospray source with an applied electrospray potential of 1.5 kV and capillary temperature of 160 °C. The LIT-MS was operated in a data-dependent mode where each full MS scan was followed by five MS/MS scans, in which the five most abundant peptide molecular ions detected from the MS scan were dynamically selected for five subsequent MS/MS scans using a collisional-induced dissociation (CID) energy of 35%. Dynamic exclusion was utilized to minimize redundant MS/MS acquisition.

The CID spectra were analyzed using SEQUEST operating on a 40 node Beowulf parallel virtual machine cluster computer (ThermoElectron) and the *Homo sapiens* proteome database at the Expert Protein Analysis System (ExPASy) website on the world wide web. Only peptides with conventional tryptic termini (allowing for up to two internal missed cleavages) possessing delta-correlation scores (DCn) > 0.08 and charge state-dependent cross-correlation (Xcorr) criteria, as follows, were considered as legitimate identifications: >1.9 for [M+H]¹⁺, >2.2 for [M+2H]²⁺, >3.1 for [M+3H]³⁺ peptide molecular ions.

### Western Blots

Solubilized high affinity APF-binding bladder epithelial cell proteins were prepared as described above. Solubilized bladder epithelial cell membrane proteins were prepared by rinsing cells in the culture flasks with ice cold PBS, then scraping the cells into ice-cold extraction buffer (20mM Tris/HCL, pH 7.4, containing 50 µg/ml PMSF, 2 µg/ml aprotinin, 100 µM tosyl-lysine-chloromethyl-ketone, 1 µM pepstatin A, and 1 µg/ml diisopropylfluorophosphate). The cell slurry was then vortexed until homogeneous, and centrifuged at 4 °C for 10 minutes at 14,000 rpm. Supernatant was removed, and the pelleted membrane fraction was washed once with ice cold extraction buffer, repelleted, and proteins were solubilized using ice cold extraction buffer containing 1% Triton X-100. Solubilized total cell proteins were extracted into ice cold RIPA buffer containing 1.0% NP40, as previously described (Keay *et al*., 2003)*.*

Protein concentration of solubilized protein samples was measured using a Folin reagent-based protein assay kit (Bio-Rad). The solubilized proteins were then incubated for 10 minutes at 70 °C in sample reducing buffer, each lane was loaded with 20 µg protein, and proteins separated by electrophoresis using 4-12% NuPAGE Novex Bis-Tris polyacrylamide gels (InVitrogen) in MOPS-SDS running buffer (InVitrogen) according to the manufacturer's instructions, and transferred to nitrocellulose membranes (InVitrogen) according to the NuPAGE gel manufacturer's protocol for Western transfer (30 V constant voltage for 1 hour). Following protein transfer, the nitrocellulose membranes were blocked with 5% nonfat dry milk in TBS-T buffer (Tris-buffered saline, pH 7.4, with 0.1% Tween 20), and incubated overnight at 4°C in TBS-T buffer containing mouse monoclonal anti-CKAP4/p63 antibodies ("anti-CLIMP-63", clone G1/296) (Alexis Biochemicals), mouse monoclonal anti-vimentin antibodies (BD Bioscience), or mouse monoclonal anti-β-actin antibodies (Sigma); when more than one antibody was used for binding to proteins on a single membrane, the membrane was stripped between antibody incubations using Re-Blot Plus Mild solution (Chemicon) according to the manufacturer's instructions. The membranes were subsequently washed three times with TBS-T, incubated with horseradish peroxidase-conjugated goat anti-mouse IgG secondary antibodies (Santa Cruz Biotechnology) for 1 hour at room temperature, and developed with ECL Chemiluminiscence Reagent (Amersham Biosciences).

### ³H-Thymidine Incorporation

Cell proliferation was measured by ³H-thymidine incorporation into explanted normal human bladder epithelial cells, as previously described *(Keay et al.,* 2004; Keay *et al.,* 1996). Briefly, purified lyophilized synthetic APF was resuspended in acetonitrile/distilled water (1:1), diluted in serum-free MEM (containing only glutamine and antibiotics/antimycotics), and applied to the cells; cell controls received acetonitrile/distilled water diluted in serum-free MEM alone. Cells were then incubated at 37°C in a 5% CO₂ atmosphere for 48 hours. The cell contents harvested and methanol-fixed onto glass fiber filter paper, and the amount of radioactivity incorporated determined. Significant inhibition of 3H-thymidine incorporation was defined as a mean decrease in counts per minute of greater than 2 standard deviations from the mean of control cells for each plate.

### Rhodamine-Labeled Synthetic APF

Rhodamine-APF was synthesized in a similar manner to the biotinylated APF using solid phase methods, starting with N-α-Fmoc-N-ε(ivDde)-L-lysinyl 2-chlorotrityl resin. After coupling of the sugar-threonine residue to the peptide, the glycopeptide was treated with hydrazine hydrate (1:11 in MeOH) to remove the sugar protecting groups and also the lysine ivDde protecting group. The glycopeptide was cleaved from the resin and purified by HPLC on a C₈ column. It was then dissolved in DMF, and 5(6)-Rhodamine B isothiocyanate (Sigma-Aldrich) (1 equivalent) and triethylamine (3 equivalents) were added. The solution was left stirring in the dark for 1½ hours. After removal of the solvent, the residue was dried under vacuum overnight and purified by HPLC. The identity of the rhodamine-labelled synthetic APF was confirmed by mass spectrometry.

### Confocal Immunofluorescence Microscopy

Normal control bladder epithelial cells were plated at a density of 2 x 10⁴ cells/well on 8-well LabTek chamber slides (Nalge Nunc International, Naperville, IL), and grown to confluence in DMEM-F12 containing 10% heat-inactivated FBS, 1% antibiotic/antimycotic solution, 1% L-glutamine, 0.25 units/ml insulin, and 5 ng/ml rhEGF. On day 2, the medium was changed to serum-free MEM medium containing 1% antibiotic/antimycotic solution and 1% L-glutamine. After 48 hours, the cells were fixed using ethanol/acetone (1:1) for 15 min at room temperature, washed three times with 1 x PBS, and incubated with monoclonal anti-CKAP4 antibody 1:100 mouse monoclonal anti-CKAP4/p63 antibodies ("anti-CLIMP-63", clone G1/296) (Alexis Biochemicals) diluted in PBS, for 2 hrs at 37 °C. Cells were then washed three times with PBS and further incubated with FITC-labelled secondary antibody diluted in PBS [goat anti-Mouse IgG 1:2000 (Zymed)] for 2 hrs at 37 °C. Following five additional washes with PBS, cells were incubated with rhodamine-labeled synthetic APF diluted in PBS at room temperature for 30 minutes, washed with PBS, and examined using a Zeiss LSM510 confocal laser-scanning microscope. Negative controls for the method included cells incubated without primary and/or secondary antibodies or APF, as well as cells incubated with secondary antibody alone.

### siRNA

Double stranded siRNA corresponding to nucleotides 594-616 (5'-AACUUUUGAGUCCAUCUUGAGAA - 3' sense strand; SEQ ID NO:5) of CKAP4/p63 (NM_006825) and a scrambled double stranded negative control siRNA (5'-AAUUCUGUAUGCUA CCUGUAGAA - 3' sense strand; SEQ ID NO:13) were prepared by preincubating single-stranded sense and anti-sense strands prepared with double A overhangs in serum-free MEM medium at 37°C for 1 hour. Primary normal bladder epithelial cells were trypsinized for 10 minutes at room temperature, centrifuged in growth medium (MEM with 10% heat-inactivated FBS, 1% antibiotic/antimycotic solution, and 1% L-glutamine), and the cell pellet was resuspended in serum-free medium at a density of 1 x 10⁶ cells/ml. 200 µl of the cell suspension was then transferred to a sterile 2 mm cuvette with 14 µg siRNA, and electroporated at 160 V/500 µF capacitance using a BioRad Gene Pulser Xcell. The cells were then immediately transferred to culture flasks (for Western blot) or 96 well plates (for thymidine incorporation assay).

### REFERENCES

All patents, patent applications, and publications mentioned in the specifications are indicative of the levels of those skilled in the art to which the invention pertains.

### PATENTS AND PATENT APPLICATIONS

U.S. Patent 5,440,013

U.S. Patent 5,618,914

U.S. Patent 5,670,155

U.S. Patent 5,446,128

U.S. Patent 5,710,245

U.S. Patent 5,840,833

U.S. Patent 5,859,184

U.S. Patent 5,929,237

U.S. Patent 5,475,085

U.S. Patent 5,672,681

U.S. Patent 5,674,976

U.S. Patent 4,554,101

Johnson *et al.,* 1993

### PUBLICATIONS

Alaiya, A., Al-Mohanna, M., and Linder, S. (2005) J. Proteome Res. 4: 1213-1222.

Auger, G., Blanot, D., van Heijenoort, J., Nadal, C., Gournay, M. F., Winchenne, J. J., Boffa, G. A., Lambin, P., Maes, P., and Tartar, A. (1989) J Cell Biochem 40, 439-451.

Banham, A.H., Turley, H., Pulford, K., Gatter, K., and Mason, D.Y. (1997) J. Clin. Pathol. 50:485-489.

Danahey, D.G., Wu, J.C., Lin, L.H., and DePhilip, R.M. (1995) J. Urol. 154: 2190-2196.

Fattaey, H.K., Betz, N.A., Westhoff, B.A., Moos, P.J., and Johnson, T.C. (1997) Breast Cancer Res. Treat. 42: 125-136.

Gillenwater, J.Y., and Wein, A.J. (1987) J. Urol. 140: 203-206, 1987.

Held, P.J., Hanno, P.M., Wein, A.J., Pauly, M.V., and Cann, M.A. (1990) Interstitial Cystitis. London: Springer-Verlag, pp. 29-48.

Keay, S., Kleinberg, M., Zhang, C-O., Hise, M.K., and Warren, J.W. (2000) J. Urol. 64: 2112-2118.

Keay, S., Seillier-Moiseiwitsch, F., Zhang, C-O., Chai, T.C., and Zhang, J. (2003) Physiological Genomics 14: 107-115.

Keay, S., Warren, J.W., Zhang, C-O, Tu, L.M., Gordon, D.A., and Whitmore, K.E. (1999) J. Urol. 162: 1487-1489.

Keay, S., Zhang, C-O, Hise, M,, Trifillis, A.L., Hebel, J.R., Jacobs, S.C., and Warren, J.W. (1996) J. Urol. 156: 2073-2078.

Keay, S., Zhang, C-O, Kagen, D.I., Hise, M.K., Jacobs, S.C., Hebel, J.R., Gordon, D., Whitmore, K., Bodison, S., and Warren, J.W. (1997) J. Urol. 158: 1983-1988.

Keay, S., Zhang, C-O, Shoenfelt, J., Erickson, D.R., Whitmore, K., Warren, J.W., Marvel, R., and Chai T. (2001) Urology 57 (6 Suppl 1): 9-14.

Keay, S., Zhang, C-O., Shoenfelt, J.L., and Chai, T.C. (2003) Urology 61: 1278-1284.

Keay, S.K., Szekely Z., Conrads T.P., Veenstra T.D., Barchi J.J., Jr., Zhang C-O, Koch K.R., and Michejda C.J. (2004) Proc. Natl. Acad. Sci., USA 101:11803-11808.

Klopfenstein, D.R., Kappeler, F., and Hauri, H-P. (1998) EMBO J. 17: 6168-6177.

Matthews, Y.L., Abele, S.T., Kusek, J.W., Nyberg, L.M., and the Interstitial Cystitis Database Study Group. (2001) Urology 57 (Suppl 6A): 67-81.

Okazaki, Y., Ohno, H., Takase, K., Ochiai, T., and Saito, T. (2000) J. Biol. Chem. 275: 35751-35758.

Parsons, C.L., Lilly, J.D., and Stein, P. (1991) J. Urol. 145: 732-735.

Razzaq, T.M. et al. Functional regulation of tissue plasminogen activator on the surface of vascular smooth muscle cells by the type-II transmembrane protein p63 (CKAP4). J. Biol. Chem. 278: 42679-42685, 2003.

Schweizer, A., Rohrer, J., Slot, J.W., Geuze, H.J., and Kornfeld, S. (1995) J. Cell Sci. 108: 2477-2485.

Skoluda, D., Wegner, K., and Lemmel, E-M. (1974) Urologe 13: 15-23.

Slobodov, G., Feloney, M., Gran, C., Kyker, K.D., Hurst, R.E., and Culkin, D.J. (2004) J. Urol. 171: 1554-1558.

Tran, H., Pankov R, Tran SD, Hampton B, Burgess WH, Yamada KM. (2002) J. Cell Sci. 115: 2031-2040.

Vedrenne, C., Klopfenstein, D.R., and Hauri, H-P. (2005) Mol. Biol. Cell 16: 1928-1937.

Wilm, M. Shevchenko A, Houthaeve T, Breit S, Schweigerer L, Fotsis T, Mann M. (1996) Nature 379: 466-469.

Zhang, C., Wang, J., Koch, K., and Keay, S. (2005) J. Urol. 174: 2382-2387.

Zhou, M., Conrads, T.P., and Veenstra, T.D. (2005) Brief Funct. Genomic Proteomic 4: 69-75.

Although the present invention and its advantages have been described in detail, it should be understood that various changes, substitutions and alterations can be made herein . Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A method of screening for a modulator of the binding of APF to cytoskeletal associated protein 4 (CKAP4), comprising:
(a) providing a candidate modulator;
(b) admixing the candidate modulator with APF, or an antiproliferative congener of APF, and CKAP4;
(c) measuring binding of APF to CKAP4; and
(d) comparing the binding measured in step (c) with the binding in the absence of said candidate modulator, wherein a difference indicates that said candidate modulator is a modulator of the binding of APF to CKAP4.

2. The method of claim 1, wherein the modulator enhances binding of APF to CKAP4.

3. The method of claim 1, wherein the modulator inhibits binding of APF to CKAP4.

4. The method of claim 1, wherein the binding of the candidate modulator to CKAP4 is measured in step (c).

5. The method of any one of the preceding claims, further comprising formulating said modulator for delivery to an individual in need thereof.

6. The method of claim 5, wherein the individual in need thereof is further defined as an individual with interstitial cystitis and the modulator is an inhibitor of the binding of APF to the CKAP4.

7. The method of claim 5, wherein the individual in need thereof has cancer or has at least one risk factor for developing bladder cancer or kidney cancer and wherein the modulator enhances binding of APF to CKAP4.

8. The method of claim 1, further comprising the step of assaying said candidate modulator for cell growth inhibition activity.

## Patentansprüche

1. Screeningverfahren für einen Modulator der Bindung von APF an Cytoskelett-assoziiertes Protein 4 (CKAP4), umfassend:
(a) Bereitstellen eines Kandidaten-Modulators;
(b) Vermischen des Kandidaten-Modulators mit APF oder einem antiproliferativen Artverwandten von APF und CKAP4;
(c) Messen einer Bindung von APF an CKAP4 und
(d) Vergleichen der in Schritt (c) gemessenen Bindung mit der Bindung in Abwesenheit des Kandidaten-Modulators, wobei eine Differenz anzeigt, dass der Kandidaten-Modulator ein Modulator der Bindung von APF an CKAP4 ist.

2. Verfahren gemäß Anspruch 1, wobei der Modulator eine Bindung von APF an CKAP4 verstärkt.

3. Verfahren gemäß Anspruch 1, wobei der Modulator eine Bindung von APF an CKAP4 inhibiert.

4. Verfahren gemäß Anspruch 1, wobei die Bindung des Kandidaten-Modulators an CKAP4 in Schritt (c) gemessen wird.

5. Verfahren gemäß einem der vorangehenden Ansprüche, das außerdem Formulieren des Modulators zur Abgabe an ein Individuum, das Bedarf dafür hat, umfasst.

6. Verfahren gemäß Anspruch 5, wobei das Individuum, das Bedarf dafür hat, weiter als ein Individuum mit interstitieller Cystitis definiert wird und der Modulator ein Inhibitor der Bindung von APF an CKAP4 ist.

7. Verfahren gemäß Anspruch 5, wobei das Individuum, das Bedarf dafür hat, Krebs hat oder wenigstens einen Risikofaktor für die Entwicklung von Blasenkrebs oder Nierenkrebs hat und wobei der Modulator eine Bindung von APF an CKAP4 verstärkt.

8. Verfahren gemäß Anspruch 1, das außerdem den Schritt des Analysierens des Kandidaten-Modulators bezüglich Zellwachstumsinhibierungsaktivität umfasst.

## Revendications

1. Procédé de recherche par criblage d'un modulateur de la liaison du facteur APF à la protéine CKAP4 (protéine 4 associée au cytosquelette), lequel procédé comporte les étapes suivantes :
a) prendre un candidat modulateur ;
b) mélanger ce candidat modulateur avec de l'APF, ou avec un congénère antiprolifératif de l'APF, et avec de la CKAP4 ;
c) mesurer la liaison de l'APF à la CKAP4 ;
d) et comparer la liaison mesurée dans l'étape (c) avec la liaison observée en l'absence dudit candidat modulateur, étant entendu qu'une différence indique que ledit candidat modulateur est bien un modulateur de la liaison de l'APF à la CKAP4.

2. Procédé conforme à la revendication 1, dans lequel le modulateur promeut la liaison de l'APF à la CKAP4.

3. Procédé conforme à la revendication 1, dans lequel le modulateur inhibe la liaison de l'APF à la CKAP4.

4. Procédé conforme à la revendication 1, dans lequel on mesure, dans l'étape (c), la liaison du candidat modulateur à la CKAP4.

5. Procédé conforme à l'une des revendications précédentes, qui comporte en outre le fait de formuler ledit modulateur pour l'administrer à un individu qui en a besoin.

6. Procédé conforme à la revendication 5, dans lequel l'individu qui en a besoin est plus précisément défini comme étant un individu atteint de cystite interstitielle, et le modulateur est un inhibiteur de la liaison de l'APF à la CKAP4.

7. Procédé conforme à la revendication 5, dans lequel l'individu qui en a besoin est atteint d'un cancer ou présente au moins un facteur de risque de développement d'un cancer de la vessie ou d'un cancer du rein, et le modulateur est un promoteur de la liaison de l'APF à la CKAP4.

8. Procédé conforme à la revendication 1, qui comporte en outre une étape où l'on évalue l'activité d'inhibition de la prolifération cellulaire dudit candidat modulateur.
